# EUROPEAN PATENT SPECIFICATION

(11) **EP 1 567 647 B1**
(45) Date of publication and mention of the grant of the patent: **03.10.2007**
(21) Application number: 03775599.8
(22) Date of filing: 17.11.2003
(51) Int. Cl.: C12N 15/62, C12Q 1/37, C07K 14/47, C07K 14/245

(54) **SOLUBLE NOTCH-BASED SUBSTRATES FOR GAMMA SECRETASE AND METHODS AND COMPOSITIONS FOR USING SAME**
LÖSLICHE NOTCH BASIERTE SUBSTRATE FÜR GAMMA SECRETASE, METHODEN, ZUSAMMENSETZUNGEN UND VERWENDUNGEN DAVON
SUBSTRATS NOTCH SOLUBLES POUR SECRETASE GAMMA, ET PROCEDES ET COMPOSITIONS POUR L'UTILISATION DE CEUX-CI

(30) Priority: 26.11.2002 US 429206 P
(43) Date of publication of application: 31.08.2005
(73) Proprietor: Pharmacia & Upjohn Company LLC, Kalamazoo, MI 49001 (US)
(72) Inventor: RANK, Kenneth, Bruce, Mattawan, MI 49071 (US); SHARMA, Satish, Kumar, Ann Arbor, MI 48105 (US)
(74) Representative: Walls, Alan James
(86) International application number: PCT/IB2003/005233
(87) International publication number: WO 2004/048578

(56) References cited:
- WO-A-01/62897
- PETIT A ET AL: "NEW PROTEASE INHIBITORS PREVENT GAMMA-SECRETASE-MEDIATED PRODUCTIONOF ABETA40/42 WITHOUT AFFECTING NOTCH CLEAVAGE" NATURE CELL BIOLOGY, MACMILLAN PUBLISHERS, GB, vol. 3, no. 5, May 2001 (2001-05), pages 507-511, XP001015215 ISSN: 1465-7392
- KARLSTROM HELENA ET AL: "A sensitive and quantitative assay for measuring cleavage of presenilin substrates" JOURNAL OF BIOLOGICAL CHEMISTRY, AMERICAN SOCIETY OF BIOLOGICAL CHEMISTS, BALTIMORE, MD, US, vol. 277, no. 9, 1 March 2002 (2002-03-01), pages 6763-6766, XP002231028 ISSN: 0021-9258
- OLSON R E ET AL: "Progress towards testing the amyloid hypothesis: inhibitors of APP processing." CURRENT OPINION IN DRUG DISCOVERY & DEVELOPMENT. ENGLAND JUL 2001, vol. 4, no. 4, July 2001 (2001-07), pages 390-401, XP008028676 ISSN: 1367-6733 cited in the application
- WOLFE M S ET AL: "THE ROLE OF PRESENILINS IN GAMMA-SECRETASE ACTIVITY" JOURNAL OF BIOLOGICAL CHEMISTRY, AMERICAN SOCIETY OF BIOLOGICAL CHEMISTS, BALTIMORE, MD, US, vol. 276, no. 8, 23 February 2001 (2001-02-23), pages 5413-5416, XP001010514 ISSN: 0021-9258
- DAVIS GREGORY D ET AL: "New fusion protein systems designed to give soluble expression in Escherichia coli" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 65, no. 4, 1999, pages 382-388, XP002192026 ISSN: 0006-3592

## Description

### Field of the Invention

The present invention is directed to novel soluble substrates for γ-secretase. More particularly, the invention provides a soluble fusion polypeptide with a Notch segment containing the γ-secretase-dependent cleavage sites (γ and ε) fused to a NusA protein. Methods and compositions for making and using such a fusion protein are disclosed.

### Background

Alzheimer's disease (AD) causes progressive dementia with consequent formation of amyloid plaques, neurofibrillary tangles, gliosis and neuronal loss. The disease occurs in both genetic and sporadic forms whose clinical course and pathological features are quite similar. Three genes have been discovered to date which, when mutated, cause an autosomal dominant form of AD. These encode the amyloid protein precursor (APP) and two proteins, presenilin-1 (PS 1) and presenilin-2 (PS2), which are structurally and functionally related. Mutations in any of the three proteins have been observed to enhance proteolytic processing of APP via an intracellular pathway that produces amyloid beta peptide (Aβ peptide, sometimes referred to as Abeta), a 40-42 amino acid peptide that is the primary component of amyloid plaque in AD (Younkin, Brain Pathol. 1(4):253-62, 1991; Haass, J. Neurosci. 11 (12):3783-93, 1991).

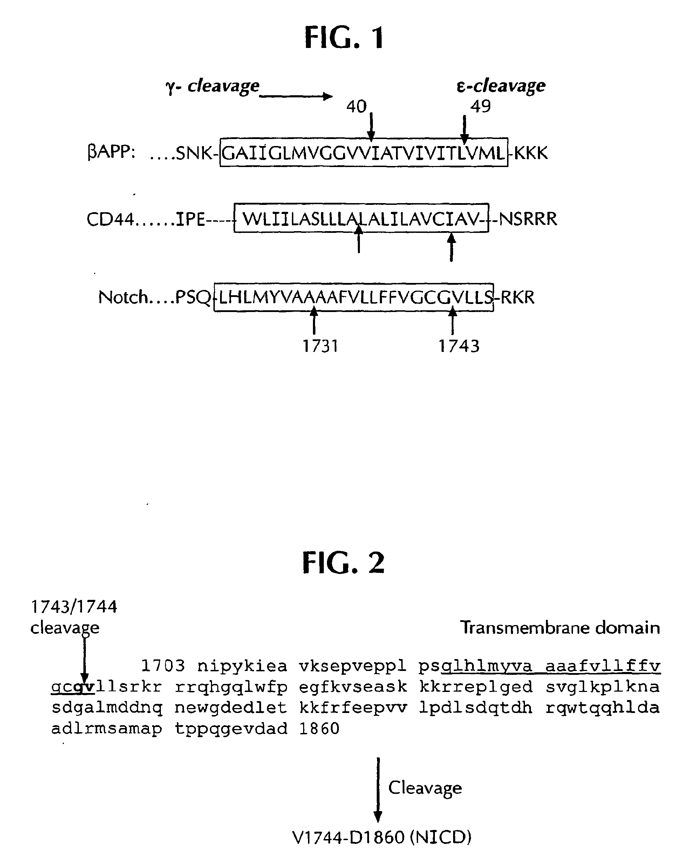

Dysregulation of intracellular pathways for proteolytic processing may be central to the pathophysiology of AD. In the case of plaque formation, mutations in APP, PS1 or PS2 consistently alter the proteolytic processing of APP so as to enhance formation of Aβ 1-42, a form of the Aβ peptide which seems to be particularly amyloidogenic, and thus very important in AD. APP localizes to the secretory membrane structure including the cell surface, and has a transmembrane domain near the C-terminus (FIG. 1). Examples of specific isotypes of APP which are currently known to exist in humans include the 695-amino acid polypeptide described by Kang et al. (1987), Nature 325: 733-736 which is designated as the "normal" APP; the 751 amino acid polypeptide described by Ponte et al. (1988), Nature, 331: 525-527 (1988) and Tanzi et al. (1988), Nature, 331: 528-530; and the 770 amino acid polypeptide described by Kitaguchi et. al., Nature, 331: 530-532 (1988).

The Aβ peptide is derived from a region of APP adjacent to and containing a portion of the transmembrane domain (see FIG. 1). Normally, processing of APP at the α-secretase site cleaves the midregion of the Aβ sequence adjacent to the membrane and releases the soluble, extracellular domain of APP from the cell surface. This α-secretase APP processing creates soluble APP-α, which is not thought to contribute to AD. However, pathological processing of APP at the β- and γ-secretase sites, which are located N-terminal and C-terminal to the α-secretase site, releases the Aβ peptide. The β-secretase cleavage site is located 28 residues from the plasma membrane luminal surface and the APP γ-secretase cleavage site is located in the transmembrane region. Processing at the β- and γ-secretase sites can occur in both the endoplasmic reticulum (in neurons) and in the endosomal/lysosomal pathway after reinternalization of cell surface APP (in all cells).

Thus, the enzymatic activities of the β- and γ-secretase enzymes are targets for drug discovery (Olson et al., Curr. Opin. Drug Discovery & Develop. 4:390-401, 2001). These two enzymes act in concert to cleave APP, which is cleaved initially by β-secretase to produce membrane-bound C-terminal (CT) fragment called CT-100, which in turn serves as a substrate for the membrane-associated γ-secretase. The intramembrane-cleavage of CT-100 by presenilin 1(PS1)-dependent γ-secretase results in the production of Aβ 1-40 and 1-42. In addition, there is another cleavage event (termed gamma-like or epsilon-secretase cleavage) cleaves near residue 721 of APP at approximately 2-5 residues inside the cytoplasmic membrane boundary to generate a series of stable, C-terminal APP fragments (FIG. 1).

Notch-1 belongs to the Notch family of cell surface receptors, which play a widespread role in the assignation of cell fates. In recent years, it has been postulated that APP processing is similar to the processing of the cell surface receptor Notch 1 (Wolfe et al., J. Biol. Chem. 276:5413-5416, 2001). Indeed, it has been shown that APP and Notch-1 are competitive substrates for the putative endogenous γ-secretase. Notch-1 is an integral-membrane protein that is proteolytically processed within its ectodomain upon ligand-mediated activation. Following ligand binding, Notch-1 undergoes presendin-dependent intramembraneous γ-secretase cleavages (Okochi, EMBO J. 2 5408-5416, 2002). The first is at 1731/1732 site (this is akin to the Aβ-like γ secretase cleavage) and the second is at the 1743/1744 site (this is akin to the ε cleavage in the generation of APP and is sometimes referred to as S3-cleavage of Notch; depicted in FIG. 1 as an "ε" cleavage). It is the ε cleavage at 1743/1744 junction of Notch that occurs toward the end of the transmembrane domain to release the Notch 1 intracellular domain (NICD). The released NICD translocates to the nucleus, where it interacts with a DNA binding protein denoted CSL (this acronym stands for three separate names given to this protein in different systems: CBF1/RBP-J in mammals, Suppressor of Hairless [Su(H)] in *Drosophila* and *Xenopus;* and Lag-1 in *C. Elegans*). The complex formed between NICD and CSL modifies the transcription of target genes. NICD is required for signaling pathway critical in embryonic development (Schroeter 1998; Hupert 2000).

Presemlin-dependent γ-secretase activity is required for processing of the Notch receptor to NICD (De Stropper et al., Nature 398:518-522,1999). It has been reported that, in cells, Notch 1 and APP are competitive substrates for PS1-dependent γ-secretase cleavage (Berezovska et al., J. Biol. Chem. 276:30018-30023,2001). As such, γ-secretase inhibitors designed to inhibit the production of pathogenic Aβ also inhibit Notch signaling. This has significant implications for the potential use of γ-secretase inhibitors as drugs in the treatment ofAD. The inhibition of Notch-1 processing in the adult would lead to immunodeficiency and anemia because of the important function of Notch-1 in hematopoesis. Thus, there is a need to identify compounds that specifically inhibit APP CT-100 cleavage but do not inhibit Notch cleavage. Such compounds would serve as therapeutic agents for the intervention of AD without producing deleterious effects of inhibition of NICD production.

Petit et al, Nat Cell Biol (2001) May; 3(5): 507-11 describe non-peptidic p inhibitors of γ-secretase and also cell based assays which take into account presenilin cleavage of myc-tagged Notch.

Karlstrom et al (JBC Vol 277, No 9, pp6763-6766) describe a quantitative assay for γ-secretase-mediated cleavage of Alzheimer amyloid precursor protein (APP) using a C99 form of APP in which a Gal4 DNA-binding/VP16 transactivation domain has been incorporated.

### Summary of the Invention

The preset invention provides methods and compositions for identifying compounds that do not inhibit γ-secretase mediated cleavage of Notch. These methods and compositions circumvent the problem of inhibition of NICD production that attends non-specific inhibition of γ-secretase inhibition. The present invention therefore allows for the identification of therapeutic agents for the intervention of AD without producing deleterious effects of inhibition of NICD production.

A first aspect of the present invention provides a soluble fusion protein comprising recombinant Notch protein fused to the C--terminus of a NusA protein sequence. Preferably the Notch protein comprises the transmembrane domain of Notch protein. However, the Notch protein may contain more or less of the full-length Notch protein than the transmembrane domain so long as the Notch protein contains the S3 cleavage site of Notch. The Notch protein for a specific embodiment comprises the sequence of SEQ ID NO:4 and is encoded by a nucleic acid sequence of SEQ ID NO:3. The recombinant Notch protein should be one which comprises the S3 cleavage site (i.e., the ε cleavage site) of Notch. The recombinant Notch protein may be a vertebrate Notch protein or an invertebrate Notch protein. In certain embodiments, the recombinant Notch protein is derived from mouse Notch protein having the sequence of SEQ ID NO:5 [Gen Bank accession number Z11886]. More particular embodiments, contemplate the use of a recombinant Notch protein comprises amino acids 1703 through 1860 of mouse Notch protein. Any of the soluble fusion proteins of the present invention may further comprise a C-terminal His-tag and/or a C-terminal Flag-tag. Of course, all or a portion of a human Notch sequence, e.g., the sequence of SEQ ID NO:6 [Genbank accession number M73980] also could be used.

The present invention further contemplates polynucleotides comprising a nucleotide sequence that encodes a fusion proteins described herein. An exemplary polynucleotide sequence that encodes such a fusion protein is one which comprises a sequence set forth in SEQ ID NO: 1. This polynucleotide sequence encodes a fusion protein of SEQ ID NO:2. Also contemplated herein is an expression vector comprising a polynucleotide of the present invention. The expression vector preferably is one in which the polynucleotide is operably linked to a promoter to promote expression of the protein encoded by the polynucleotide in a host cell.

Recombinant host cells transformed or transfected with a polynucleotide or expression vector described herein also are encompassed by the present invention. The invention contemplates a method of producing a substrate for a γ-secretase assay comprising growing such a recombinant host cell in a manner allowing expression of the fusion protein. The method may further comprise purifying the polypeptide. In such a method, the host cell may be any host cell amenable to recombinant protein production including a mammalian host cell, a bacterial host cell and a yeast host cell. In exemplary embodiments, the host cell is a Hela cell, a human embryonic kidney cell line 293 cell, a fibroblast, or a CHO cell.

Another aspect of the present invention provides a method of producing a solubilized Notch protein, the method comprising preparing a fusion protein wherein the Notch protein is fused to the C-terminus of a NusA protein. More specifically, the method comprises a recombinant production of the fusion protein, which involves preparing an expression construct comprising a nucleic acid that encodes a fusion protein comprising a Notch protein containing the amino acids of the S3 cleavage site of Notch linked at the C-terminus of a NusA protein; transforming a host cell with the expression construct under conditions that facilitate the expression of the fusion protein; and growing the transformed host cell in culture. The method further may comprise isolating the fusion protein from the transformed host in culture. In certain embodiments, the method comprises producing the fusion protein through chemical protein synthesis. In such methods, the Notch protein preferably comprises amino acids 1703 through 1860 of mouse Notch protein.

Also contemplated herein is an *in vitro* method of assaying for γ-secretase mediated ε cleavage (1743/1744) of Notch protein comprising contacting a first composition comprising a mammalian γ-secretase complex or biologically active fragment thereof, with a second compositions comprising a fusion protein of the invention; and measuring cleavage of the fusion protein.

The γ-secretase complex of the above method may comprise a membrane fraction purified and isolated from mammalian cells or cells transformed or transfected with expression constructs comprising nucleotide sequences that encode the γ-secretase complex. In the above method, the fusion protein is a solubilized Notch protein prepared according to the methods discussed herein.

The invention specifically contemplates compositions comprising γ-secretase modulators identified through the screening methods described herein. Also encompassed by the present invention is a method of modulating γ-secretase activity *in vivo* comprising a step of administering a modulator identified by the screening methods described herein that is a γ-secretase modulator that is selective for inhibiting γ-secretase-mediated cleavage of APP as compared to γ-secretase-mediated cleavage of Notch protein to a mammal in an amount effective to modulate γ-secretase activity in cells of the mammal.

The present invention also is directed to pharmaceutical compositions comprising one or more modulators identified by the present invention and a pharmaceutically acceptable carrier. Also contemplated is a method of treating a disease or condition characterized by an abnormal γ-secretase activity comprising administering to a subject in need of treatment a pharmaceutical composition of the invention. Use of the modulators identified herein in the manufacture of a medicament for the treatment of Alzheimer's Disease is particularly contemplated.

Other features and advantages of the present invention will become apparent from the following detailed description. It should be understood, however, that the detailed description and the specific examples, while indicating preferred embodiments of the invention, are given by way of illustration only, because various changes and modifications within the scope of the invention will become apparent to those skilled in the art from this detailed description.

### Brief Description of the Drawings

The following drawings form part of the present specification and are included to further illustrate aspects of the present invention. The invention may be better understood by reference to the drawings in combination with the detailed description of the specific embodiments presented herein.
Figure 1. Comparison of γ-secretase-like cleavage sites. The sequence surrounding the transmembrane domains of APP (SBQ ID NO:10), Notch-1 (SBQ ID NO:12), and B-cathedrin (SEQ ID NO:11) are shown. Also shown are the γ-secretase cleavage sites in APP to produce Aβ 1-40 and 1-42 as well as the ε-cleavage (ε) or γ-secretase-like cleavage sites for the three substrates.
Figure 2. Amino acid sequence surrounding the γ-secretase-like cleavage site in Notch-1. This sequence (1703-1860; SEQ ID NO:13) was chosen for expression in *E. coli.* The transmembrane domain is indicated by underlining and the 1743/1744 cleavage site is indicated in bold and underlined characters. Cleavage of this Notch sequence would result in a NICD fragment containing amino acids 1744-1860.

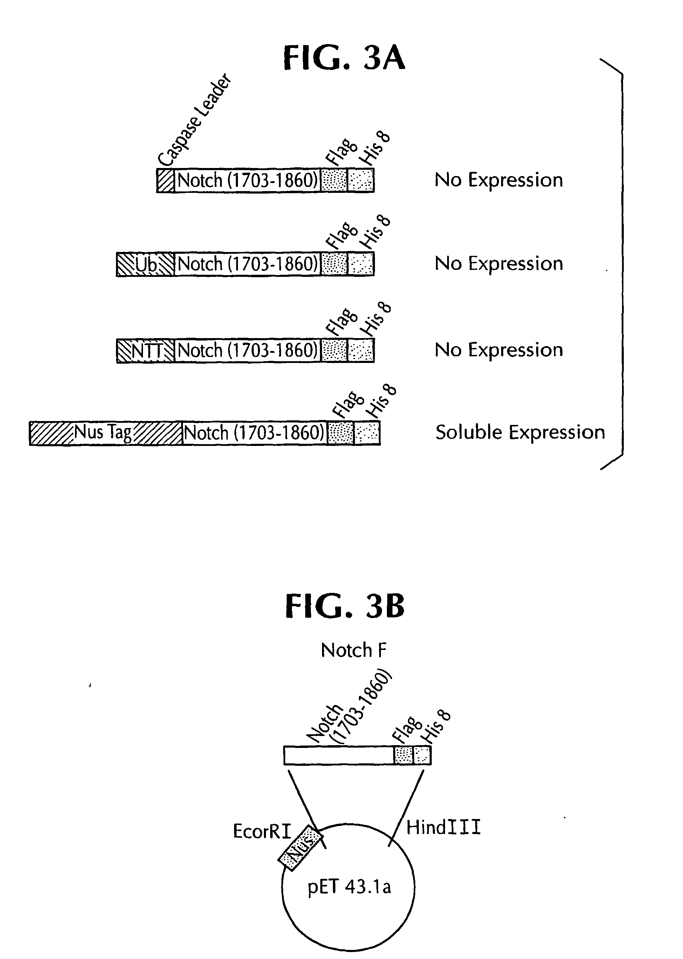

Figure 3. a) Constructs cloned for expression of Notch in *E. coli.* Four constructs are shown, all containing the amino acid sequence 1703-1860 of Notch-1. No expression was seen when either a caspase leader sequence, Ubiquitin, or the N-terminal domain of tau was used as an N-terminal tag to help drive expression. When Notch (1703-1860) was fused to the NusA protein, soluble expression in *E. coli* was seen. b) A schematic of the Notch F construct cloned into pET 43.1a. This construct also contains C-terminal Flag and 8His tags.

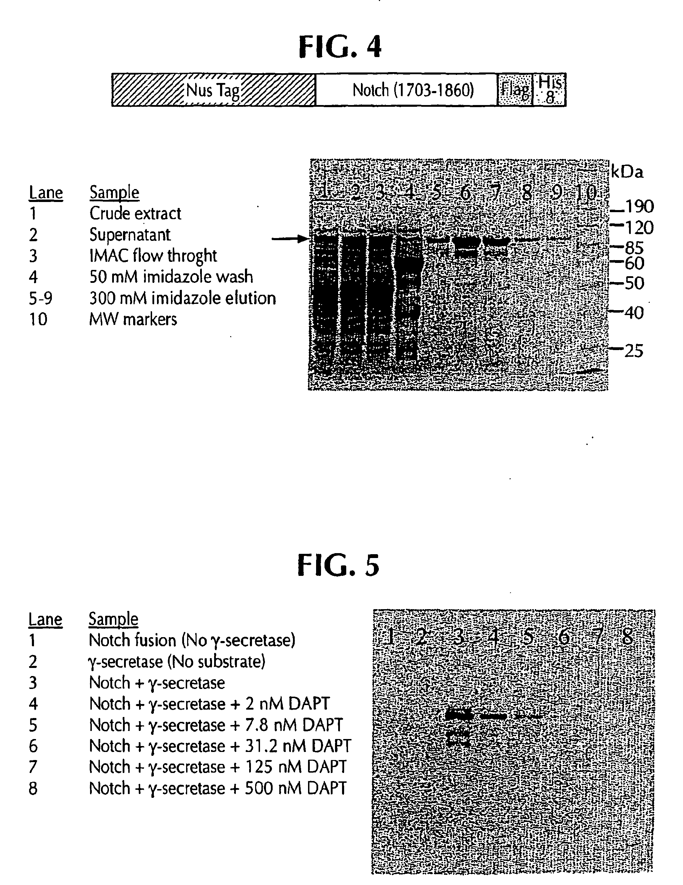

Figure 4. IMAC isolation of NusA-Notch fusion. A schematic of the fusion protein is shown at the top. Details of expression and purification of NusA-Notch can be found in the Materials and Methods. The arrow indicates the fusion protein expression band on the 10 % SDS-PAGE gel. Lane 1, crude *E. coli* lysate; lane 2, *E. coli* supernatant, lane 3, IMAC flow through; lane 4, 50 mM imidazole wash; lanes 5-9, 300 mM imidazole elution fractions; lane 10, BenchMark molecular weight marker.
Figure 5. Western blot showing the cleavage of NusA-Notch fusion using solubilized γ-secretase. The details of the experiment are in Example 1. Briefly, NusA-Notch fusion was incubated overnight with solubilized γ-secretase in the presence and absence of varying concentrations of DAPT (PHA-568638). The samples were electrophoresed, blotted and probed with Val1744 antibody, specific for cleavage at Val 1744.

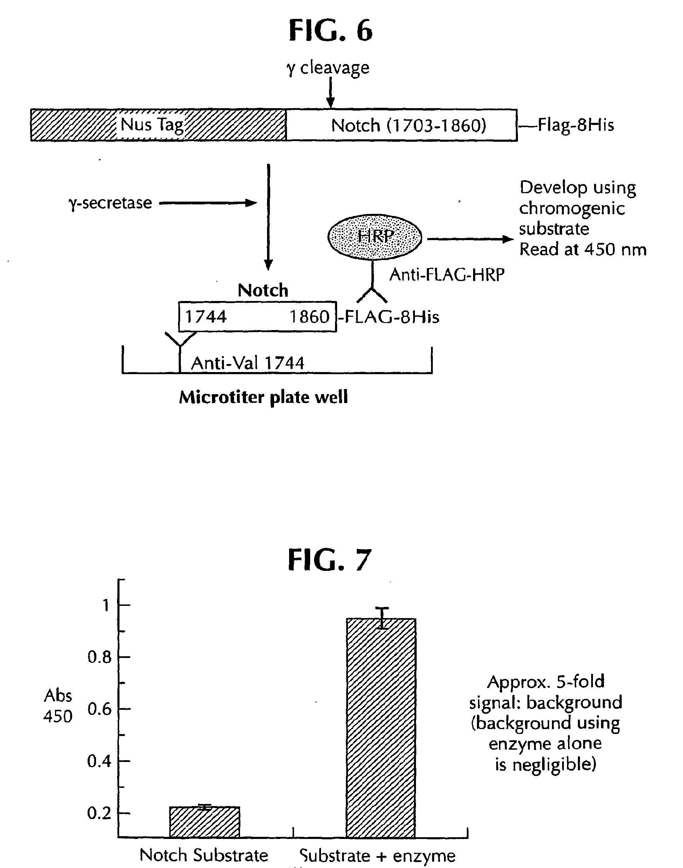

Figure 6. Detection of specific notch cleavage by ELISA. A schematic of the sandwich ELISA used to detect cleavage of the NusA-Notch fusion by solubilized γ-secretase is shown. Specific cleavage is detected using the Val1744 antibody.
Figure 7. Cleavage of NusA-Notch fusion protein by γ-secretase as detected by ELISA. NusA-Notch (0.9 µM) substrate was incubated in the absence or presence of 68 µg/ml solubilized γ-secretase (enzyme) and the ELISA done according to the Materials and Methods. The data represents the average of six experiments.

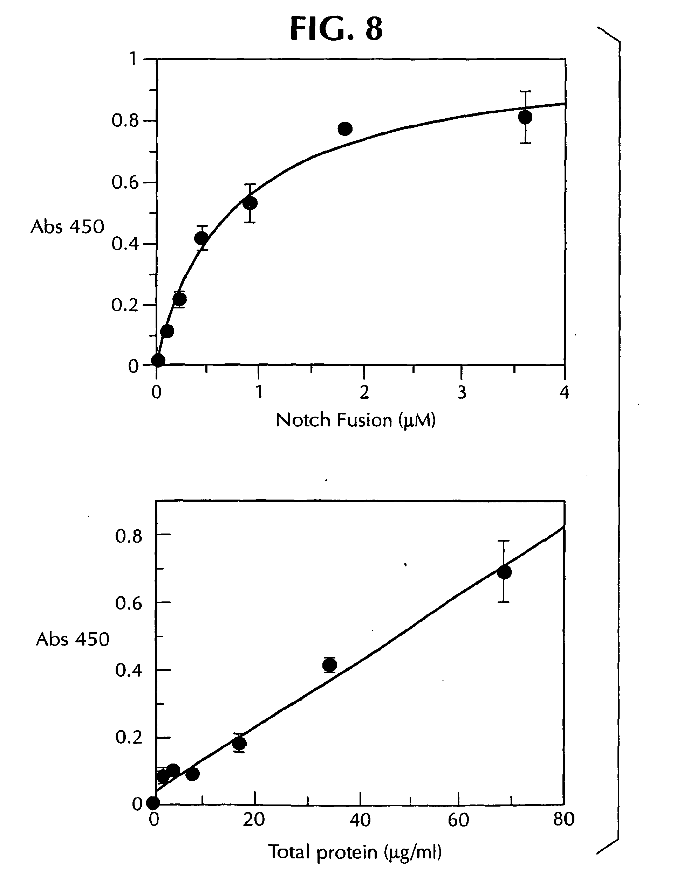

Figure 8. Characterization of notch cleavage by ELISA. The ELISA was performed as described in the Materials and Methods. When varying the Notch substrate concentration, 0.11 to 3.6 µM of NusA-Notch was incubated with 68 µg/ml solubilized γ-secretase and the data plotted using an Michaelis-Menton curve fit. When the enzyme concentration was varied, 2.1 to 68 µg/ml solubilized γ-secretase was incubated with 0.9 µM Notch substrate. The data represent the average of three experiments and were plotted using GraFit 4.0. There was an approx. 5-fold signal: background (background using enzyme alone is negligible).

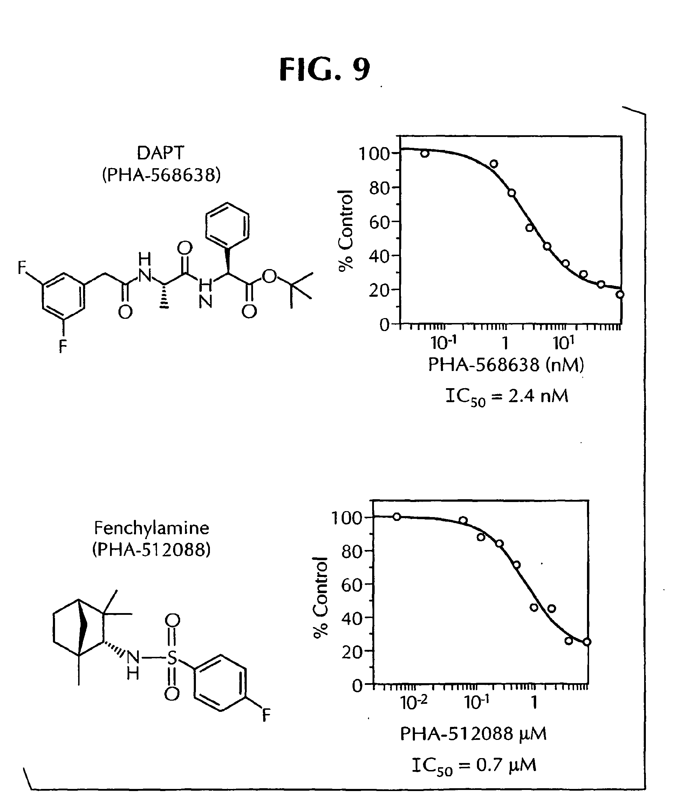

Figure 9. Inhibition of notch cleavage by compounds known to inhibit the *in vitro* cleavage of CT-100 by γ-secretase. The inhibition profiles for DAPT (PHA-568638) and fenchylamine (PHA-512088), as measured using the notch cleavage ELISA are shown. Also shown are the relative IC₅₀ values.

### Detailed Description of the Preferred Embodiments

AD is a leading age related disorder associated with progressive dementia and pathology characterized by cortical atrophy and deposition of senile plaques and neurofibrillary tangles. A primary component of the plaques is the 40-42 amino acid long peptide, Aβ, derived from a region of APP adjacent to and containing a portion of the transmembrane domain of the full length APP. This pathogenic peptide is generated as a result of sequential processing due to β- and γ-secretases activities. Thus, the enzymatic activities of these two secretase enzymes are targets for drug discovery (Olson et al., Curr. Opin. Drug Discovery & Develop. 4:390-401, 2001).

The processing of the cell surface receptor Notch has been shown to be similar to APP processing (Wolf et al., J. Biol. Chem. 276:5413-5416, 2001). Figure 1 shows a comparison of γ-secretase-like cleavage sites in APP and Notch. As shown, the cleavage site in APP is in the middle of the transmembrane domain, while Notch is cleaved very close to the C-terminal end of its transmembrane domain. A similar ε-cleavage site has been recently reported (Marambaud et al., EMBO J., 21:1948-1956, 2002) in cathedrin E (Figure 1). Figure 2 shows the amino acid sequence from 1703-1860 of Notch protein including the S3 cleavage site in Notch (Steiner et al., J. Mol. Neurosci. 17:193-198, 2001). The specific cleavage at the 1743/1744 junction would produce the V1744-D1860 Notch intracellular domain (NICD), a fragment of the NICD observed in cells transfected with mΔE Notch constructs (1704 to 2183) lacking the S1 and S2 cleavage sites (Kopan et al., Proc. Natl. Acad. Sci. 93:1683-1688, 1996). Although a Notch construct (Val 1711-Glu 1809) has been reported (Esler et al Proc. Natl. Acad. Sci. 99, 2720-2725,2002), the specific cleavage by γ-secretase at the 1743/1744 junction *in vitro* has not been described. The specific cleavage at the 1743/1744 junction can be readily determined through the use of Val-1744 antibody (Cell Signaling Technology). This antibody is specific for cleaved Notch and does not cross react with uncleaved Notch protein.

Thus, in cells, in addition to cleaving the CT-100 fragment produced by the action of the β-secretase, PS1-dependent γ-secretase also cleaves at the ε site 1743/1744 to produce NICD. This cleaved NICD translocates to the nucleus and is involved in signaling. Therapeutic inhibition of γ-secretase activity designed to alleviate AD, also results in an inhibition of NICD production. The present invention for the first time provides methods and compositions for identifying therapeutic agents which do not inhibit the production of NICD from Notch-1.

The methods of the present invention provide *in vitro* assays for Notch cleavage and the use of such assays in the secondary evaluation of γ-secretase inhibitors. These assays employ a soluble Notch substrate for γ-secretase, which comprises a soluble fusion protein comprising recombinant Notch protein fused to the C--terminus of a NusA protein sequence. The assays of the present invention may be performed as direct *in vitro* ELISA assays and/or Western blots for Notch protein cleavage by γ-secretase. The methods of the present invention provide for the production and purification of a soluble recombinant Notch protein (Asn 1703-Asp 1860) substrate expressed in a suitable cell lines, e.g., *E. coli,* as a fusion protein with NusA engineered to a Notch protein sequence (see Fig. 6).

Using the purified fusion protein substrate of the present invention, the inventors demonstrated specific cleavage at the 1743/1744 site in Notch using solubilized γ-secretase from HeLa cells. As described in the detailed Examples, the cleaved Notch protein can be detected using an antibody specific for Val-1744. The inventors validated that assay and showed that cleavage of the Notch protein was inhibited in a dose-dependent manner by DAPT, a well-known potent inhibitor of γ-secretase. An ELISA was developed based on anti-Val-1744 antibodies. Further validation of the *in vitro* Notch assay was provided by inhibition of the cleavage by γ-secretase inhibitors. Operationally, an inhibitor or a modulator is defined as compound which lowers Aβ through γ-secretase.

### I. Notch Substrate for in vitro Notch Assay

The Notch substrate for use in the assays of the present invention is a soluble fusion protein of a Notch polypeptide to a Nus protein. The fusion protein may be labeled or otherwise modified to facilitate the purification of the peptide, detection of the Notch fusion protein itself, or a detection of the cleavage product of the Notch fusion protein upon the action of the γ-secretase. Production of the fusion protein and exemplary modifications are described in further detail herein below.

In a preferred aspect of the present invention, the Notch substrate containing amino acids N1703-D1860 of mouse notch-1 protein (DNA Sequence accession number Z11886; see FIG. 2) joined at the N-terminal to the C-terminus of NusA. It is contemplated that the fusion polypeptide may be produced by recombinant protein production or indeed by automated peptide synthesis as discussed elsewhere in the specification. The transmembrane domain in FIG 2 spans from amino acid 1723 through to 1744 (see FIG. 1). γ-secretase cleaves Notch at the ε-cleavage between amino acids 1743 and 1744. This is cleavage also is termed the S3-cleavage site and generates NICD (i.e., a peptide spanning amino acids V1744-D1860).

In addition to this novel fusion protein, the present invention further contemplates the generation terminal additions, also called fusion proteins or fusion polypeptides, of the Notch/NusA fusion protein substrate described above or identified according to the present invention. Moreover, while the preferred embodiments of the present invention show a Notch/NusA fusion peptide comprising N1703-D1860 of mouse Notch-1, it should be understood that the Notch protein may be derived from any source. Such a source may be mammalian, or non-mammalian. Thus, while it is preferred that the Notch-1 is derived from human, mouse, rat or another mammalian source, it is contemplated that in certain embodiments, the Notch-1 may be derived from *e.g., C. elegans, Xenopus, drosophila,* and other invertebrate sources.

Furthermore, it is contemplated that any Notch-1 derivative that contains the γ-secretase cleavage sites 1731/1732 (γ site) and 1743/1744 site (ε cleavage) will be useful in the fusion protein substrate of the present invention. In Notch-1, the γ-secretase cleavage site that releases NICD is located 1743/1744 site in Notch. It is contemplated that the distance between the cleavage site and the start of a NusA is about 500 amino acids in order to mimic the steric properties of the Notch γ-secretase cleavage domain. This distance may be generated from the NusA protein or it may be created by means of a heterologous peptide linker. Preferably, this region is from the NusA protein. The NusA protein domain component of the Notch/NusA fusion polypeptide may be essentially any portion of the NusA protein that allows the Notch/NusA fusion to remain soluble and therefore amenable to an *in vitro* assay.

NusA has previously been used to produce the soluble expression of proteins in *E. coli.* (See e.g., Wilkinson, et aL, Bio/Technology 9, 443-448, 1991; Davis et al., Biotechnol Bioeng., 65(4):382-8, 1999). Herein, a NusA protein comprising the sequence of SEQ ID NO:17 (encoded by a nucleic acid sequence of SEQ ID NO:16) is fused to Notch. However, those of skill in the art may employ a NusA sequence other than the sequence depicted in SEQ ID NO:17 and still produce a soluble Notch/NusA fusion protein of the present invention. For example, one of skill may use a NusA protein comprising 80%, 85%, 90%, 95%, 96%, 97%, 98% or more sequence homology with the sequence of SEQ ID NO:17. Alternatively, those of skill may employ a smaller contiguous fragment NusA derived from SEQ ID NO: 17, for example the fragment may be 50, 100, 150, 200, 250, 300, 350, 400, 425, 450, 475, 500, 510, 520, 530, 540, 550 or more contiguous amino acids of SEQ ID NO: 17.

General principles for designing and making fusion proteins are well known to those of skill in the art. For example, fusions typically employ leader sequences from other species to permit the recombinant expression of a protein or peptide in a heterologous host. Another useful fusion includes the addition of an immunologically active domain, such as an antibody epitope, to facilitate purification of the fusion polypeptide. Inclusion of a cleavage site at or near the fusion junction will facilitate removal of the extraneous polypeptide after purification. The recombinant production of these fusions is described in further detail elsewhere in the specification. Other useful fusions include linking of functional domains, such as active sites from enzymes, glycosylation domains, cellular targeting signals or transmembrane regions.

More particularly, the present invention contemplates a fusion polypeptide in which there is a first component comprising the Notch protein containing the 1731/1732 (γ cleavage) and 1743/1744 (ε cleavage) cleavage sites attached to a second component comprising all or a portion of, the NusA protein. In additional embodiments, the fusion polypeptide further may comprise a third component which comprises a reporter gene product. In still further embodiments, the fusion polypeptides may further comprise a tagged sequence component. A particular fusion polypeptide that is contemplated is one which comprises a reporter gene product on one side of a NusA portion, a stretch of sequence containing the Notch protein with the γ-secretase cleavage sites, and tagged sequence on the other side of the Notch protein. The reporter gene product used in the fusion polypeptides of the present invention may be any reporter protein commonly used by those of skill in the art. Exemplary reporter proteins include but are not limited to luciferase; secreted alkaline phosphatase (SEAP); β galactosidase; β- glucoronidase; green fluorescent protein and chloramphenical acetyl transferase.

Other particular embodiments further contemplate a tagged sequence as a fourth component of the fusion polypeptides of the present invention. There are various commercially available fusion protein expression systems that may be used to provide a tagged sequence in this context of the present invention. Particularly useful systems include but are not limited to the glutathione S-transferase (GST) system (Pharmacia, Piscataway, NJ), the maltose binding protein system (NEB, Beverley, MA), the FLAG system (IBI, New Haven, CT), and the 6xHis system (Qiagen, Chatsworth, CA). These systems are capable of producing recombinant polypeptides bearing only a small number of additional amino acids, which are unlikely to affect the biologically relevant activity of the recombinant fusion protein. For example, both the FLAG system and the 6xHis system add only short sequences, both of which are known to be poorly antigenic and which do not adversely affect folding of the polypeptide to its native conformation. Another N terminal fusion that is contemplated to be useful is the fusion of a Met Lys dipeptide at the N terminal region of the protein or peptides. Such a fusion may produce beneficial increases in protein expression and/or activity. Specific tagged sequences that are contemplated for use in the present invention include the C-terminal FLAG tag sequence DYKDDDDK (SEQ ID NO: 14). In addition, the tagged sequences also may contain an 8His tag to produce a FLAG/8his tag attached to the fusion protein (DYKDDDDKHHHHHHHH, SEQ ID NO:15).

An example of a preferred fusion protein of the present invention is one in which NusA is fused to either a partial or a full-length mouse Notch-1 protein that comprises the 1731/1732 and 1743/1744 γ-secretase cleavage sites together with a short C-terminal FLAG/8His-tagged tail. The sequence of an exemplary fusion polypeptide is comprises amino acids 1703-1860 of mouse Notch (*i.e*., depicted in SEQ ID NO:13) fused to a NusA protein. This exemplary fusion polypeptide has the sequence of SEQ ID NO:2. In order to monitor cleavage of this chimeric construct by γ-secretase, anti-Val 1744 antibodies against the Notch cleavage product containing Val-1744 residue are employed to determine the presence and concentration of the Val-1744 fragment generated as a result of Notch cleavage. Anti-Flag antibodies may be employed to detect the C-terminus of the chimeric construct. However, it should be noted that the chimeric construct may also employ a reporter protein such as alkaline phosphatase, for example, at the C-terminus instead of the flag tag. Such a reporter protein would be released as a result of the Notch cleavage and detected using assays well known to those of skill in the art.

In addition to providing fusion polypeptides as already described, the invention provides Notch fusion proteins that are further modified to incorporate, for example, a label or other detectable moiety.

For example, the Notch/NusA fusion protein substrates may comprise internally quenched labels that result in increased detectability after cleavage of the Notch substrate. The Notch/NusA fusion protein substrates may be modified to have attached a paired flurophore and quencher including but not limited to 7-amino, 4-methyl coumarin and 2,4-dinitrophenol, respectively, such that cleavage of the peptide by the γ-secretase results in increased fluorescence due to physical separation of the flurophore and quencher, which are attached on opposite sides of the scissile bond for the γ-secretase. Other paired flurophores and quenchers include bodipy-tetramethylrhodamine and QSY-5 (Molecular Probes, Inc.) In a variant of this assay, biotin or another suitable tag may be placed on one end of the peptide to anchor the peptide to a substrate assay plate and a flurophore may be placed at the other end of the fusion protein. Useful flurophores include those listed above as well as Europium labels such as W8044 (EG&g Wallac, Inc.) Another preferred label that may be used is Oregon green that may be attached to a Cys residue. Cleavage of the fusion protein by γ-secretase will release the flurophore or other tag from the plate, allowing compounds to be assayed for inhibition of proteolytic cleavage as shown by an increase in retained fluorescence. Preferred colorimetric assays of γ-secretase proteolytic activity utilize other Notch/NusA fusion proteins in which the amino acids comprising the γ-secretase recognition site for cleavage are linked to o-nitrophenol through an amide linkage, such that cleavage of the fusion protein by the γ-secretase results in an increase in optical density after altering the assay buffer to alkaline pH.

Further, the Notch/NusA fusion proteins may be labeled using labels well known to those of skill in the art, *e.g.*, biotin labels are particularly contemplated. The use of such labels is well known to those of skill in the art and is described in, *e.g.*, U.S. No. Patent 3,817,837; U.S. Patent No. 3,850,752; U.S. Patent No. 3,996,345 and U.S. Patent No. 4,277,437. Other labels that will be useful include but are not limited to radioactive labels, fluorescent labels and chemiluminescent labels. U.S. Patents concerning use of such labels include, for example, U.S. Patent No. 3,817,837; U.S. Patent No. 3,850,752; U.S. Patent No. 3,939,350 and U.S. Patent No. 3,996,345. Any of Notch/NusA fusion protein compositions of the present invention may comprise one, two, or more of any of these labels.

### II. γ-Secretase Compositions

In addition to novel Notch fusion proteins, the present invention is directed to methods of using such Notch fusion proteins in various γ-secretase assays. The present section provides a discussion of generating fractions containing proteins that have aγ- secretase activity.

While the exact identity of γ-secretase remains elusive there is strong evidence that γ-secretase may be presenilin 1. Regardless of the fact that the sequence of the γ-secretase protein has yet to be identified, those of skill in the art are aware of methods and compositions for isolating cellular fractions that comprises γ-secretase. For example, Li et al. (Proc. Natl. Acad. Sci. 97:6138-6143, 2000) described methods and compositions for producing a membrane preparation which contains a solubilized γ-secretase activity. Such a method is useful in the present invention for providing a purified fraction containing a γ-secretase. Once such a fraction is produced, it is contemplated that it may be used in the assays of the present invention. In addition, the novel fusion proteins of the present invention also may be used to further isolate and purify the γ-secretase from such a membrane fraction using, *e.g.*, affinity chromatographic separation techniques.

The γ-secretase fraction generally is isolated from any cell that expresses a γ-secretase activity. For example, HeLa3 cells may be used. The cells are ruptured using e.g., a French press or other cell rupture technique, including but not limited to, freeze-thaw techniques (*e.g.*, cycling cells between dry ice and 37°C water bath); solid shear methods using a Hughes or French press; detergent lysis (*e.g.*, on-ionic detergent solutions such as Tween, Triton, NP-40, *etc*.); hypotonic solution lysis (*e.g.*, water, citric buffer); liquid shear methods (homogenizer, impinging-jet microfluidizer); sonication (ultrasound). After cell lysis, the cell debris and nuclei can be removed by sedimentation using centrifugation. The membrane fraction is precipitated at *e.g.*, 100,000g for 60 minutes and the membrane fraction may be further solubilized using detergent. For example, in the solubilization process taught by Li et al supra, the membrane fraction pellet from the 100,000g centrifugation step is resuspended in buffer and treated with 1% CHAPSO for 60 minutes at 4°C and centrifuged for 60 minutes. This detergent solubilization process is such that the supernatant of the 100,000g fraction contains the solubdized γ-secretase.

The above solubilized fraction is used in the γ-secretase assays described herein throughout.

### III. Protein or Peptide Production and Purification

The present invention provides soluble Notch protein substrates for use in the identification of modulators of γ-secretase that are specific for APP cleavage but do not inhibit the cleavage of Notch. Such substrates may be produced by conventional automated peptide synthesis methods or by recombinant expression.

### A. Synthetic Peptide Production

The peptides or indeed even the full length fusion polypeptides of the invention can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co., (1984) ; Tam et al., J. Am. Chem. Soc., 105:6442, 1983; Merrifield, Science, 232:341-347, 1986; and Barany and Merrifield, The Peptides, Gross and Meienhofer, eds, Academic Press, New York, 1-284, 1979, each incorporated herein by reference. The novel Notch fusion protein substrates of the invention comprise the γ-secretase cleavage sites 1731/1732 and 1743/1744 that are amenable to cleavage by γ-secretase can be readily synthesized and then screened in γ-secretase screening assays.

In particularly preferred methods, the fusion proteins of the present invention were synthesized by solid-phase technology employing a Model 433A from Applied Biosystems Inc. The purity of any given Notch fusion protein, generated through automated peptide synthesis or through recombinant methods may be determined using reverse phase HPLC analysis. Chemical authenticity of each peptide may be established by any method well known to those of skill in the art. In preferred embodiments, the authenticity is established by mass spectrometry.

Additionally, the fusion proteins may be quantitated using amino acid analysis in which microwave hydrolyses are conducted. Such analyses may use a microwave oven such as the CEM Corporation's MDS 2000 microwave oven. The peptide (approximately 2 mg protein) is contacted with 6 N HCl (Pierce Constant Boiling e.g., about 4 ml) with approximately 0.5% (volume to volume) phenol (Mallinckrodt). Prior to the hydrolysis, the samples are alternately evacuated and flushed with N2. The protein hydrolysis is conducted using a two-stage process. During the first stage, the fusion proteins are subjected to a reaction temperature of about 100 °C and held that temperature for 1 minute. Immediately after this step, the temperature is increased to 150 °C and held at that temperature for about 25 minutes. After cooling, the samples are dried and amino acid from the hydrolysed fusion proteins samples are derivatized using 6-aminoquinolyl-N-hydroxysuccinimidyl carbamate to yield stable ureas that fluoresce at 395 nm (Waters AccQ·Tag Chemistry Package). The samples may be analyzed by reverse phase HPLC and quantification may be achieved using an enhanced integrator.

### B. Recombinant Protein Production

As an alternative to automated peptide synthesis, recombinant DNA technology may be employed wherein a nucleotide sequence which encodes a peptide of the invention is inserted into an expression vector, transformed or transfected into an appropriate host cell and cultivated under conditions suitable for expression as described herein below. Recombinant methods are especially preferred for producing longer polypeptides that comprise peptide sequences of the invention.

From the disclosure of novel Notch fusion protein substrates of the present invention, it is possible to produce the fusion polypeptides by recombinant techniques. A variety of expression vector/host systems may be utilized to contain and express the peptide or fusion polypeptide coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the Notch fusion proteins in bacteria, yeast and other invertebrates are described herein below.

Expression vectors for use in prokaryotic hosts generally comprise one or more phenotypic selectable marker genes. Such genes generally encode, e.g., a protein that confers antibiotic resistance or that supplies an auxotrophic requirement. A wide variety of such vectors are readily available from commercial sources. Examples include pSPORT vectors, pGEM vectors (Promega), pPROEX vectors (LTI, Bethesda, MD), Bluescript vectors (Stratagene), pET vectors (Novagen) and pQE vectors (Qiagen). The DNA sequence encoding the given Notch fusion protein is amplified by PCR and cloned into such a vector, for example, pGEX-3X (Pharmacia, Piscataway, NJ) designed to produce a fusion protein comprising glutathione-S-transferase (GST), encoded by the vector, and a protein encoded by a DNA fragment inserted into the vector's cloning site. The primers for the PCR may be generated to include for example, an appropriate cleavage site. Treatment of the recombinant fusion protein with thrombin or factor Xa (Pharmacia, Piscataway, NJ) is expected to cleave the fusion protein, releasing the substrate or substrate containing polypeptide from the GST portion. The pGEX-3X/fusion peptide construct is transformed into E. coli XL-1 I Blue cells (Stratagene, La Jolla CA), and individual transformants were isolated and grown. Plasmid DNA from individual transformants is purified and partially sequenced using an automated sequencer to confirm the presence of the desired peptide or polypeptide encoding nucleic acid insert in the proper orientation.

The induction of the GST/substrate fusion protein is achieved by growing the transformed XL-1 Blue culture at 37°C in LB medium (supplemented with carbenicillin) to an optical density at wavelength 600 nm of 0.4, followed by further incubation for 4 hours in the presence of 0.5 mM Isopropyl *-D-Thiogalactopyranoside (Sigma Chemical Co., St. Louis MO).

The GST fusion protein, expected to be produced as an insoluble inclusion body in the bacteria, may be purified as follows. Cells are harvested by centrifugation; washed in 0.15 M NaCl, 10 mM Tris, pH 8, 1 mM EDTA; and treated with 0.1 mg/ml lysozyme (Sigma Chemical Co.) for 15 minutes at room temperature. The lysate is cleared by sonication, and cell debris is pelleted by centrifugation for 10 minutes at 12,000 X g. The fusion protein-containing pellet is resuspended in 50 mM Tris, pH 8, and 10 mM EDTA, layered over 50% glycerol, and centrifuged for 30 min. at 6000 X g. The pellet is resuspended in standard phosphate buffered saline solution (PBS) free of Mg++ and Ca++. The fusion protein is further purified by fractionating the resuspended pellet in a denaturing SDS polyacrylamide gel (Sambrook *et al.* eds. Cold Spring Harbor Press, Cold Spring Harbor, N.Y. 1989). The gel is soaked in 0.4 M KCl to visualize the protein, which is excised and electroeluted in gel-running buffer lacking SDS. If the GST/Notch fusion protein is produced in bacteria as a soluble protein, it may be purified using the GST Purification Module (Pharmacia Biotech).

The fusion protein may be subjected to thrombin digestion to cleave the GST from the mature Notch fusion polypeptide. The digestion reaction (20-40 µg fusion protein, 20-30 units human thrombin (4000 U/mg (Sigma) in 0.5 ml PBS) is incubated 16-48 hrs. at room temperature and loaded on a denaturing SDS-PAGE gel to fractionate the reaction products. The gel is soaked in 0.4 M KCl to visualize the protein bands. The identity of the protein band corresponding to the expected molecular weight of the fusion polypeptide may be confirmed by partial amino acid sequence analysis using an automated sequencer (Applied Biosystems Model 473A, Foster City, CA).

Alternatively, the DNA sequence encoding the predicted substrate containing fusion polypeptide may be cloned into a plasmid containing a desired promoter and, optionally, a leader sequence (see, e.g., Better et al., Science, 240:1041-43, 1988). The sequence of this construct may be confirmed by automated sequencing. The plasmid is then transformed into *E. coli* using standard procedures employing CaCl₂ incubation and heat shock treatment of the bacteria (Sambrook *et al., supra*). The transformed bacteria are grown in LB medium supplemented with carbenicillin, and production of the expressed protein is induced by growth in a suitable medium. If present, the leader sequence will effect secretion of the mature Notch-based fusion protein and be cleaved during secretion.

The secreted recombinant protein is purified from the bacterial culture media by the method described herein throughout.

Similarly, yeast host cells from genera including Saccharomyces, Pichia, and Kluveromyces may be employed to generate the recombinant peptide. Preferred yeast hosts are *S. cerevisiae* and *P. pastoris.* Yeast vectors will often contain an origin of replication sequence from a 2T yeast plasmid, an autonomously replicating sequence (ARS), a promoter region, sequences for polyadenylation, sequences for transcription termination, and a selectable marker gene. Vectors replicable in both yeast and *E. coli* (termed shuttle vectors) may also be used In addition to the above-mentioned features of yeast vectors, a shuttle vector will also include sequences for replication and selection in *E. coli.* Direct secretion of polypeptides expressed in yeast hosts may be accomplished by the inclusion of nucleotide sequence encoding the yeast I-factor leader sequence at the 5' end of the substrate-encoding nucleotide sequence.

Generally, a given substrate may be recombinantly expressed in yeast using a commercially available expression system, e.g., the Pichia Expression System (Invitrogen, San Diego, CA), following the manufacturer's instructions. This system also relies on the pre-pro-alpha sequence to direct secretion, but transcription of the insert is driven by the alcohol oxidase (AOX1) promoter upon induction by methanol.

The secreted recombinant substrate is purified from the yeast growth medium by, e.g., the methods used to purify substrate from bacterial and mammalian cell supernatants.

Alternatively, a synthetic DNA encoding the novel substrate of the invention may be cloned into the baculovirus expression vector pVL1393 (PharMingen, San Diego, CA; Luckow and Summers, Bio/Technology 6:47 (1988)). This substrate-containing vector is then used according to the manufacturer's directions (PharMingen) to infect Spodoptera frugiperda cells in sF9 protein-free media and to produce recombinant protein. The protein or peptide is purified and concentrated from the media using a heparin-Sepharose column (Pharmacia, Piscataway, NJ) and sequential molecular sizing columns (Amicon, Beverly, MA), and resuspended in PBS. SDS-PAGE analysis shows a single band and confirms the size of the protein, and Edman sequencing on a Porton 2090 Peptide Sequencer confirms its N-terminal sequence.

Alternatively, the Notch fusion protein substrate may be expressed in an insect system. Insect systems for protein expression are well known to those of skill in the art. In one such system, *Autographa californica* nuclear polyhedrosis virus (AcNPV) is used as a vector to express foreign genes in *Spodoptera frugiperda* cells or in *Trichoplusia* larvae. The substrate coding sequence is cloned into a nonessential region of the virus, such as the polyhedrin gene, and placed under control of the polyhedrin promoter. Successful insertion of substrate will render the polyhedrin gene inactive and produce recombinant virus lacking coat protein coat. The recombinant viruses are then used to infect *S. frugiperda* cells or *Trichoplusia* larvae in which the substrate is expressed (Smith et al., J Virol 46:584, 1983; Engelhard EK et al., Proc. Nat. Acad. Sci. 91:3224-7, 1994).

Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

It is preferable that the transformed cells are used for long-term, high-yield protein production and as such stable expression is desirable. Once such cells are transformed with vectors that contain selectable markers along with the desired expression cassette, the cells may be allowed to grow for 1-2 days in an enriched media before they are switched to selective media. The selectable marker is designed to confer resistance to selection and its presence allows growth and recovery of cells which successfully express the introduced sequences. Resistant clumps of stably transformed cells can be proliferated using tissue culture techniques appropriate to the cell.

A number of selection systems may be used to recover the cells that have been transformed for recombinant protein production. Such selection systems include, but are not limited to, HSV thymidine kinase, hypoxanthine-guanine phosphoribosyltransferase and adenine phosphoribosyltransferase genes, in tk-, hgprt-or aprt- cells, respectively. Also, anti-metabolite resistance can be used as the basis of selection for dhfr, which confers resistance to methotrexate; gpt that confers resistance to mycophenolic acid; neo that confers resistance to the aminoglycoside G418; ALS which confers resistance to chlorsulfuron; and hygro that confers resistance to hygromycin. Additional selectable genes that may be useful include trpB, which allows cells to utilize indole in place of tryptophan, or hisD, which allows cells to utilize histinol in place of histidine. Markers that give a visual indication for identification of transformants include anthocyanins, b-glucuronidase and its substrate, GUS, and luciferase and its substrate, luciferin.

### C. Site-Specific Mutagenesis

Site-specific mutagenesis is another technique useful in the preparation of individual γ-secretase substrate peptide and more particularly fusion polypeptides that comprise as a component one of the γ-secretase substrate fusion proteins of the present invention. This technique employs specific mutagenesis of the underlying DNA (that encodes the amino acid sequence that is targeted for modification). The technique further provides a ready ability to prepare and test sequence variants, incorporating one or more of the foregoing considerations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to 25 nucleotides in length is preferred, with about 5 to 10 residues on both sides of the junction of the sequence being altered.

The technique typically employs a bacteriophage vector that exists in both a single stranded and double stranded form Typical vectors useful in site-directed mutagenesis include vectors such as the M13 phage. These phage vectors are commercially available and their use is generally well known to those skilled in the art. Double stranded plasmids also are routinely employed in site directed mutagenesis, which eliminates the step of transferring the gene of interest from a phage to a plasmid.

In general, site-directed mutagenesis is performed by first obtaining a single-stranded vector, or melting of two strands of a double stranded vector which includes within its sequence a DNA sequence encoding the desired protein. An oligonucleotide primer bearing the desired mutated sequence is synthetically prepared. This primer is then annealed with the single-stranded DNA preparation, taking into account the degree of mismatch when selecting hybridization (annealing) conditions, and subjected to DNA polymerizing enzymes such as E. coli polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as E. coli cells, and clones are selected that include recombinant vectors bearing the mutated sequence arrangement.

Of course, the above described approach for site-directed mutagenesis is not the only method of generating potentially useful mutant peptide species and as such is not meant to be limiting. The present invention also contemplates other methods of achieving mutagenesis such as for example, treating the recombinant vectors carrying the gene of interest mutagenic agents, such as hydroxylamine, to obtain sequence variants.

### D. Protein Purification

It will be desirable to purify the fusion proteins of the present invention. Protein purification techniques are well known to those of skill in the art. These techniques involve, at one level, the crude fractionation of the cellular milieu to polypeptide and non-polypeptide fractions. Having separated the peptide or polypeptides of the invention from other proteins, the fusion polypeptides or peptides of interest may be further purified using chromatographic and electrophoretic techniques to achieve partial or complete purification (or purification to homogeneity). Analytical methods particularly suited to the preparation of a pure peptide are ionexchange chromatography, exclusion chromatography, polyacrylamide gel electrophoresis; isoelectric focusing. A particularly efficient method of purifying fusion proteins is fast protein liquid chromatography (FPLC) or even high performance liquid chromatography (HPLC). In particularly preferred embodiments, the NusA-Notch fusion was isolated using immobilized metal affinity chromatography (IMAC).

IMAC is used primarily in the purification of polyhistidine tagged recombinant proteins. In the present invention the C-terminus of the fusion protein comprises a polyhistidine tag, thereby allowing purification through this powerful technique. This purification relies on the natural tendency of histidine to chelate divalent metals. Placing the metal ion on a chromatographic support allows purification of the histidine tagged proteins. This is a highly efficient method that has been employed by those of skill in the art for a variety of protein purification methods. Exemplary conditions of a preferred embodiment of isolating the protein of the present invention are described in Example 1. However, it should be understood that those of skill in the art could vary the conditions and media and still achieve purification in accordance with the present invention. To this end, those of skill in the art are referred to U.S. Patent No. 4,431,546 which describes in detail methods of metal affinity chromatographic separation of biological or related substances from a mixture. The chromatographic media described in the aforementioned patent comprise binding materials which have a ligand containing at least one of the groups anthraquinone, phthalocyanine or aromatic azo, in the presence of at least one metal ion selected from the group Ca²⁺, Sr²⁺, Ba²⁺, Al³⁺, Co²⁺, Ni²⁺, Cu²⁺ or Zn²⁺. Additional media and conditions are described at e.g., http://www.affiland.com/imac/nta.htm and http://www.affiland.com/imac/pdc.htm

Certain aspects of the present invention concern the purification, and in particular embodiments, the substantial purification, of an encoded polypeptide, protein or peptide. The term "purified polypeptide, protein or peptide" as used herein, is intended to refer to a composition, isolated from other components, wherein the polypeptide, protein or peptide is purified to any degree relative to the cellular or synthesis components used to generate the protein. A purified polypeptide, protein or peptide therefore also refers to a polypeptide, protein or peptide, free from the environment in which it may naturally occur.

Generally, "purified" will refer to a polypeptide, protein or peptide composition that has been subjected to fractionation to remove various other components, and which composition substantially retains its expressed biological activity. Where the term "substantially purified" is used, this designation will refer to a composition in which the polypeptide, protein or peptide forms the major component of the composition, such as constituting about 50%, about 60%, about 70%, about 80%, about 90%, about 95% or more of the proteins in the composition.

Various methods for quantifying the degree of purification of the polypeptide, protein or peptide will be known to those of skill in the art in light of the present disclosure. These include, for example, determining the specific activity of an active fraction, or assessing the amount of polypeptides within a fraction by SDS/PAGE analysis. A preferred method for assessing the purity of a fraction is to calculate the specific activity of the fraction, to compare it to the specific activity of the initial extract, and to thus calculate the degree of purity, herein assessed by a "-fold purification number." The actual units used to represent the amount of activity will, of course, be dependent upon the particular assay technique chosen to follow the purification and whether or not the expressed polypeptide, protein or peptide exhibits a detectable activity.

Various techniques suitable for use in protein purification will be well known to those of skill in the art. These include, for example, precipitation with ammonium sulphate, PEG, antibodies and the like or by heat denaturation, followed by centrifugation; chromatography steps such as ion exchange, gel filtration, reverse phase, hydroxylapatite and affinity chromatography, isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified polypeptide, protein or peptide.

There is no general requirement that the polypeptide, protein or peptide always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed utilizing an HPLC apparatus will generally result in a greater "-fold" purification than the same technique utilizing a low pressure chromatography system Methods exhibiting a lower degree of relative purification may have advantages in total recovery of protein product, or in maintaining the activity of an expressed protein.

It is known that the migration of a polypeptide can vary, sometimes significantly, with different conditions of SDS/PAGE (Capaldi et al., Biochem. Biophys. Res. Comm., 76:425, 1977). It will therefore be appreciated that under differing electrophoresis conditions, the apparent molecular weights of purified or partially purified expression products may vary.

### IV. Expression Constructs for use in the Production of the Substrates of the Invention

In the present invention, it may be necessary to express the Notch fusion proteins of the present invention. To achieve such expression, the present invention will employ vectors comprising polynucleotide molecules for encoding the Notch fusion proteins of the present invention, as well as host cell transformed with such vectors. Such polynucleotide molecules may be joined to a vector, which generally includes a selectable marker and an origin of replication, for propagation in a host. These elements of the expression constructs used in the present invention are described in further detail herein below.

The expression vectors include DNA encoding any of the given peptide or fusion polypeptide γ-secretase substrates described above or below, operably linked to suitable transcriptional or translational regulatory sequences, such as those derived from a mammalian, microbial, viral, or insect gene. Examples of regulatory sequences include transcriptional promoters, operators, or enhancers, mRNA ribosomal binding sites, and appropriate sequences which control transcription and translation.

The terms "expression vector," "expression construct " or "expression cassette " are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the fusion polypeptides of the invention will of course depend upon the specific host cell to be used, and is within the skill of the ordinary artisan. Examples of suitable expression vectors include pcDNA3 (Invitrogen) and pSVL (Pharmacia Biotech). A preferred vector for expression in the present invention is pcDNA3.1-Hygro (Invitrogen). Expression vectors for use in mammalian host cells may include transcriptional and translational control sequences derived from viral genomes. Commonly used promoter sequences and enhancer sequences which may be used in the present invention include, but are not limited to, those derived from human cytomegalovirus (CMV), Adenovirus 2, Polyoma virus, and Simian virus 40 (SV40). Methods for the construction of mammalian expression vectors are disclosed, for example, in Okayama and Berg (Mol. Cell. Biol. 3:280, 1983); Cosman et al. (Mol. Immunol. 23:935, 1986); Cosman et al. (Nature 312:768, 1984); EP-A-0367566; and WO 91/18982.

The expression construct will comprise a nucleic acid region that encodes the particular Notch fusion proteins of the present invention. Coding regions for use in constructing such expression vectors should encode at least the γ-secretase cleavage of the fusion proteins described herein although it is contemplated that larger polypeptides may be encoded as long as one of the peptide generated comprises γ-secretase cleavage sites 1731/1732 and 1743/1744 that are amenable to cleavage by γ-secretase.

In certain aspects of the present invention, the expression construct may further comprise a selectable marker that allows for the detection of the expression of the peptide or polypeptide. Usually the inclusion of a drug selection marker aids in cloning and in the selection of transformants, for example, neomycin, puromycin, hygromycin, DHFR, zeocin and histidinol. Alternatively, enzymes such as herpes simplex virus thymidine kinase (tk) (eukaryotic), b-galactosidase, luciferase, or chloramphenicol acetyltransferase (CAT) (prokaryotic) may be employed. Immunologic markers also can be employed. For example, epitope tags such as the FLAG system (IBI, New Haven, CT), HA and the 6xHis system (Qiagen, Chatsworth, CA) may be employed. Additionally, glutathione S-transferase (GST) system (Pharmacia, Piscataway, NJ), or the maltose binding protein system (NEB, Beverley, MA) also may be used. The selectable marker employed is not believed to be important, so long as it is capable of being expressed simultaneously with the nucleic acid encoding a gene product. Further examples of selectable markers are well known to one of skill in the art. Particularly preferred selectable markers that may be employed in the present invention are neomycin resistance or a GFP marker.

Expression requires that appropriate signals be provided in the vectors. The present section includes a discussion of various regulatory elements, such as enhancers/promoters from both viral and mammalian sources that may be used to drive expression of the nucleic acids of interest in host cells. Elements designed to optimize messenger RNA stability and translatability in host cells also are defined. The conditions for the use of a number of dominant drug selection markers for establishing permanent, stable cell clones expressing the products also are provided, as is an element that links expression of the drug selection markers to expression of the mutant phenotype.

In preferred embodiments, the nucleic acid encoding the given peptide or the nucleic acid encoding a selectable marker is under transcriptional control of a promoter. A "promoter" refers to a DNA sequence recognized by the synthetic machinery of the cell, or introduced synthetic machinery, required to initiate the specific transcription of a gene.

Nucleotide sequences are operably linked when the regulatory sequence functionally relates to the DNA encoding the Notch fusion protein. Thus, a promoter nucleotide sequence is operably linked to a given DNA sequence if the promoter nucleotide sequence directs the transcription of the sequence. Similarly, the phrase "under transcriptional control" means that the promoter is in the correct location and orientation in relation to the nucleic acid to control RNA polymerase initiation and expression of the gene.

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (tk) and SV40 early transcription units. These studies, augmented by more recent work, have shown that promoters are composed of discrete functional modules, each consisting of approximately 7-20 bp of DNA, and containing one or more recognition sites for transcriptional activator or repressor proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription.

The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter.

In various embodiments, the human cytomegalovirus (CMV) immediate early gene promoter, the SV40 early promoter, the Rous sarcoma virus long terminal repeat, β-actin, rat insulin promoter, the phosphoglycerol kinase promoter and glyceraldehyde-3-phosphate dehydrogenase promoter, all of which are promoters well known and readily available to those of skill in the art, can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient for a given purpose. By employing a promoter with well known properties, the level and pattern of expression of the protein of interest following transfection or transformation can be optimized.

Selection of a promoter that is regulated in response to specific physiologic or synthetic signals can permit inducible expression of the gene product. Several inducible promoter systems are available for production of viral vectors. One such system is the ecdysone system (Invitrogen, Carlsbad, CA), which is designed to allow regulated expression of a gene of interest in mammalian cells. It consists of a tightly regulated expression mechanism that allows virtually no basal level expression of the transgene, but over 200-fold inducibility.

Another useful inducible system is the Tet-Off^{TM} or Tet-On^{TM} system (Clontech, Palo Alto, CA) originally developed by Gossen and Bujard (Gossen and Bujard, Proc. Natl. Acad. Sci. U.S.A. 15;89(12):5547-51, 1992; Gossen et al., Science, 268(5218):1766-9, 1995).

In mammalian cells, the CMV immediate early promoter if often used to provide strong transcriptional activation. Modified versions of the CMV promoter that are less potent have also been used when reduced levels of expression of the transgene are desired. Retroviral promoters such as the LTRs from MLV or MMTV are contemplated to be useful in the present invention. Other viral promoters that may be used include SV40, RSV LTR, HIV-1 and BIV-2 LTR, adenovirus promoters such as from the E1A, E2A, or MLP region, AAV LTR, cauliflower mosaic virus, HSV-TK, and avian sarcoma virus.

In some embodiments, regulatable promoters may prove useful. Such promoters include for example, those that are hormone or cytokine regulatable. Hormone regulatable promoters include MMTV, MT-1, ecdysone and RuBisco as well as other hormone regulated promoters such as those responsive to thyroid, pituitary and adrenal hormones.

Another regulatory element contemplated for use in the present invention is an enhancer. These are genetic elements that increase transcription from a promoter located at a distant position on the same molecule of DNA. Enhancers are organized much like promoters. That is, they are composed of many individual elements, each of which binds to one or more transcriptional proteins. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have a very similar modular organization. Enhancers useful in the present invention are well known to those of skill in the art and will depend on the particular expression system being employed (Scharf D et al., (1994) Results Probl Cell Differ 20: 125-62; Bittner et al., (1987) Methods in Enzymol. 153: 516-544).

Where an expression construct employs a cDNA insert, one will typically desire to include a polyadenylation signal sequence to effect proper polyadenylation of the gene transcript. Any polyadenylation signal sequence recognized by cells of the selected transgenic animal species is suitable for the practice of the invention, such as human or bovine growth hormone and SV40 polyadenylation signals.

Also contemplated as an element of the expression cassette is a terminator. These elements can serve to enhance message levels and to minimize read through from the cassette into other sequences. The termination region which is employed primarily will be one selected for convenience, since termination regions for the applications such as those contemplated by the present invention appear to be relatively interchangeable. The termination region may be native with the transcriptional initiation, may be native to the DNA sequence of interest, or may be derived for another source.

In certain embodiments of the invention, the use of internal ribosome entry site (IRES) elements is contemplated to create multigene, or polycistronic, messages. IRES elements are able to bypass the ribosome scanning model of 5' methylated Cap dependent translation and begin translation at internal sites (Pelletier and Sonenberg, Nature, 334:320-325, 1988). IRES elements from two members of the picornavirus family (poliovirus and encephalomyocarditis) have been described (Pelletier and Sonenberg, 1988 supra), as well an IRES from a mammalian message (Macejak and Sarnow, Nature, 353:90-94, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message.

Any heterologous open reading frame can be linked to IRES elements. This includes genes for secreted proteins, multi-subunit proteins, encoded by independent genes, intracellular or membrane-bound proteins and selectable markers. In this way, expression of several proteins can be simultaneously engineered into a cell with a single construct and a single selectable marker.

### V. Use of the Substrates in γ-Secretase Assays

In specific embodiments, the present invention involves assays to monitor the activity and/or function of γ-secretase and more specifically, the γ-secretase activity and/or function of γ-secretase. These assays will involve incubating in solution a γ-secretase complex (or purified polypeptide) with a suitable substrate of the present invention, using cleavage of the Notch fusion protein as a measure of γ-secretase proteolytic activity.

### A. Assay Formats

In specific embodiments, the invention relates to a method for the identification of agents that modulate the activity of human γ-secretase. An aspect of these assays is to monitor the Notch-cleaving activity of the γ-secretase at the ε cleavage site 1743/1744 in the presence and absence of the putative modulator compound or agent. For example, such a method for determining Notch cleavage would generally comprise the steps of:
(a) contacting any of the Notch/NusA fusion protein of the present invention *in vitro* with a composition comprising a γ-secretase activity;
(b) determining the cleavage of the Notch/NusA fusion protein at the γ-secretase cleavage site of Notch by said γ-secretase.

The composition comprising γ-secretase activity would generally be any isolated composition that comprises a γ-secretase activity. As such, the composition may be a membrane fraction isolated from a cell (a natural cell that has γ-secretase activity, or a recombinant host cell that has been engineered to express such an activity). Alternatively, composition may comprise an isolated and purified γ-secretase protein, substantially free of other proteins.

In order to identify modulators of the Notch cleavage activity of γ**-**secretase, the above steps (a) and (b) are carried out in the presence and absence of the candidate modulator, and the modulating activity of the modulator is assessed by comparing the Notch cleavage activity of the γ-secretase in the presence of the test agent to the activity in the absence of the test agent to identify an agent that modulates such cleavage by the γ-secretase. Any alteration in the amount or degree of Notch cleavage in the presence of the candidate modulator is an indication of an alteration of the γ-secretase activity in the presence of the test agent identifies an agent that is a modulator of the γ-secretase activity.

Agents that cause increased cleavage relative to the control (no test agent) are scored as agonists or stimulators of γ-secretase proteolytic activity, whereas agents to cause decreased cleavage at either Aβ 1-40 and/or Aβ 1-42 are scored as inhibitors. The inhibitors of γ-secretase are of special interest because inhibitors of γ-secretase activity have therapeutic and prophylactic indications for the treatment and prevention of AD or its symptoms or progression.

Because it is desirable to find test compounds that preferentially inhibits gamma secretase mediated cleavage of a APP or CT-100 compared to cleavage of a fusion protein of the invention, test compounds may be assessed to determine their ability to modulate APP cleavage in parallel or prior to utilizing the assays of the invention. Methods of measuring gamma secretase mediated APP or CT-100 cleavage are well known in the art. As an example, WO/01/83811 teaches substrates and in-vitro assays useful in measuring APP or CT-100 cleavage. WO/01/83811 teaches a gamma-secretase substrate comprising an M terminal Met (M), APP597-695 and a Flag tag and methods and conditions for its cleavage and detection of the cleavage products (M-Aβ40 and M-Aβ40).

Inhibitors that inhibit the APP/CT-100 cleavage activity at either Aβ 1-40 and/or Aβ 1-42 but do not inhibit the measured Notch cleavage activity of the γ-secretase are most preferred.

The γ-secretase may be a purified γ-secretase polypeptide or complex or biologically active fragments, analogs, or variants, thereof. In preferred embodiments, the γ-secretase is derived from a membrane fraction from a cell that exhibits γ-secretase activity. Preferably, such a membrane fraction contains all the components needed for the γ-secretase complex. Non-human orthologs of human γ-secretase also may be used in assays.

The assays of the present invention are designed to be performed with γ-secretase polypeptide in a cell free system. For example, in a cell-free system, the contacting step may be performed by mixing the γ-secretase enzyme or a membrane fraction containing that enzyme with the peptide or protein substrate of the invention, in the presence or absence of the test agent. For optimal results, the enzyme and the γ-secretase substrate preferably are substantially purified, mixed in defined and controlled quantities, and mixed in appropriate buffers that optimize enzymatic activity and/or mimic physiological conditions.

The determining step may involve a measurement of an N-terminal fragment, a C-terminal fragment, or both, or may involve measurement of another parameter indicative of cleavage. For example, the Notch-based or other γ-secretase substrate may contain a quenched label that becomes more detectable only upon cleavage to separate the label from the quenching moiety. Alternatively, the Notch-based or other γ-secretase substrate may be fixed at the N-terminal or C-terminal end to a solid support. In this arrangement, cleavage may be measured by release from the solid support of a cleavage fragment. The release may be measured by increased label in the media, or decreased label attached to the solid support. Alternatively, the release may be measured by quantitative capture of the released peptide (e.g., with an antibody).

In a preferred embodiment of the present invention, the cleaved Notch protein is detected using an antibody specific for Val-1744. In even more preferred embodiments, the cleaved Notch protein was detected using an ELISA developed based on anti-Val 1744 antibody and anti-Flag antibodies. The anti-Val 1744 antibodies are used to detect one fragment of the cleavage, whereas the anti-Flag antibodies detect the fragment (C-terminal) of the Notch protein which produced by the action of the γ-secretase enzyme. This assay is described in further detail in the

### Examples.

Of course, the above assay is only exemplary and other assays also may be used For example, the above assay may be set up in the following manner. 384-well micro-titer plates are blocked with BSA, γ-secretase enzyme and 50µM of the γ-secretase inhibitor compound to be tested are incubated for 1 hour and the reaction is initiated by the addition of Notch/NusA fusion protein substrate. In the final assay conditions, the volume is 30µl/well; 50µM compound; 15ng enzyme/well; 250nM substrate; 5% DMSO and 0.001% TWEEN-20. The assay is incubated overnight at room temperature and the reaction is terminated by the addition of Tris-HCl, pH 8.3. An aliquot containing 6.25 pmoles of substrate is removed and the cleaved and/or uncleaved substrate is captured in a streptavidin coated plate. The plate is washed 3 time and buffer is added. The capture assay is monitored by reading the fluorescence emission of the oregon green on an LJL Analyst (Ex 485/Em 530).

Another assay that may be used herein is a fluorescent polarization assay. Fluorescence polarization is a sensitive, facile and non-destructive assay that can be exploited to monitor the effects of the candidate agents on the γ-secretase-complex of the present invention. It can be used to monitor the interaction of these substrates with the γ-secretase enzyme. Under controlled conditions, fluorescence polarization measurements can reveal the extent of "molecular tumbling" of a fluorescent molecule in solution. For example, a small molecule with a compact molecular volume would be expected to tumble rapidly. If irradiated with polarized light the rapid movement of the molecule in solution would result in extensive depolarization of the light, and would yield a readout of "low" polarization value. Under the same conditions, the increased molecular volume of a large molecule or a large complex would slow the molecular rotation (tumbling) process. As a result, less polarization of the incident plane polarized light would result and a higher polarization value would be measured.

By labeling a small ligand with a fluorescent probes, changes in the fluorescence polarization resulting from the interaction of the ligand with another system component can be measured. Such a method may be applied to measure the strength of interaction between an enzyme (γ-secretase) and a fluorescent enzyme substrate.

In an exemplary fluorescence polarization assay, in pre-blocked low affinity, black plates enzyme and inhibitory/modulatory compound are incubated for 30 minutes and the reaction initiated by the addition of 150nM substrate (e.g., a fluorescently labeled Notch/NusA fusion protein of the present invention) to a final volume of 30µl/well. The plate is then incubated at room temperature for 15 minutes and the fluorescent polarization measured on an LJL Acquest (Ex 485/Em 530).

An aspect of the present invention that would be useful in isolating and characterizing the γ-secretase is contemplated by the present invention. This aspect contemplates a binding assay for detecting compounds that bind to the active site or at an allosteric site of the enzyme. For such determinations, the use of non-hydrolyzable derivatives of the Notch substrates of the present invention may be used. For example, the presence of a statine derivative at the junction of the bond to be cleaved renders the Notch of the present invention non-hydrolyzable at the cleavage site. The substrates further may be modified with the addition of an appropriate fluorescent tag e.g., BODIPY FL to facilitate detection.

A substrate of the present invention may be labeled with a fluorescent label and used to develop a fluorescence polarization binding assay for the γ-secretase. The equilibrium dissociation constant (KD) for the interaction between the enzyme and the substrate is determined by measuring fluorescence polarization changes which result from titrating the substrate with the enzyme.

To determine the KD for the interaction of a substrate of the present invention with γ-secretase, various quantities of γ-secretase may be combined with 3.1 nM fluorescent substrate and incubated at room temperature for 3 hours. Following the incubation, fluorescence polarization is determined using an LJL Analyst (96 well format) or a PanVera Beacon (single cuvette format). An exemplary assay is performed in 25 mM sodium acetate, 20% glycerol, pH 4.75. A graphic plot of the data obtained providing the polarization values on the vertical axis and the concentration of enzyme on the horizontal axis provides the binding isotherm for the determination of the KD for the interaction of the enzyme with the substrate. The data may then be analyzed using the relation Px=PF+(PB-PF)*[E]/(KD+[E]), where P=polarization value, x=sample, F=free inhibitor, B=bound inhibitor, E= γ-secretase (Fluorescence Polarization Applications Guide, 1998; Pan Vera, Madison, WI) to obtain the KD. This assay can be used to screen for compounds that bind to the active site of the enzyme or allosterically.

It will be appreciated that the activity measurements in the presence and absence of a test agent can be performed in parallel, or sequentially, in either order. Moreover, it may not be necessary to repeat the control measurements (*i.e.,* the measurements of cleavage in the absence of a test agent) in parallel with respect to every test agent, once a reliable baseline of enzymatic activity for particular reaction conditions has been obtained. Gained knowledge of the enzymatic activity of γ-secretase towards a particular substrate (e.g., the Notch compositions of the present invention or a composition derived from APP) in the absence of inhibitors can be used as the basis for performing the comparison step.

### B. Candidate Substances

As used herein the term "candidate substance" or "test substance" refers to any molecule that is capable of modulating γ-secretase activity, and preferably human γ-secretase activity. The candidate substance may be a protein or fragment thereof, a small molecule inhibitor, or even a nucleic acid molecule. It may prove to be the case that the most useful pharmacological compounds for identification through application of the screening assay will be compounds that are identified through screening large compound libraries or that are structurally related to other known modulators of APP processing, Notch processing or both. For example, U.S. Patent No. 6,448,229, incorporated herein by reference, describes a specific class of compounds that inhibit γ-secretase without affecting Notch signalling, and hence find use in the treatment or prevention of AD. Compounds such as those described in U.S. Patent No. 6,448,229 may be verified using the assays of the present invention. In addition, such compounds may serve as starting materials in rational drug design to identify additional candidate modulators for use in the present invention. Other inhibitory agents that could be used for such rational drug design include but are not limited to DAPT (PHA-568638) and fenchylamine (PHA-512088).

The candidate substances may include fragments or parts of naturally-occurring compounds or may be only found as active combinations of known compounds which are otherwise inactive. However, prior to testing of such compounds in humans or animal models, it will be necessary to test a variety of candidates to determine which have potential.

Accordingly, the candidate substance may include fragments or parts of naturally-occurring compounds or may be found as active combinations of known compounds which are otherwise inactive. Accordingly, the present invention provides screening assays to identify agents which stimulate or inhibit γ-secretase-mediated cellular APP processing preferentially over the stimulation or inhibition of Notch by that enzyme. It is proposed that compounds isolated from natural sources, such as animals, bacteria, fungi, plant sources, including leaves and bark, and marine samples may be assayed as candidates for the presence of potentially useful pharmaceutical agents.

It will be understood that the pharmaceutical agents to be screened could also be derived or synthesized from chemical compositions or man-made compounds. Thus, it is understood that the modulator identified by the present invention may be polypeptide, polynucleotide, small molecule inhibitors or any other inorganic or organic chemical compounds that may be designed through rational drug design starting from known stimulators or inhibitors of γ-secretase activity and/or APP processing.

The candidate screening assays are simple to set up and perform. Thus, in assaying for a modulator, after obtaining a cell membrane fraction comprising a functional γ-secretase (*e.g.,* a cell membrane fraction containing a solubilized γ-secretase complex), one will admix a candidate substance with such a γ-secretase composition in the presence of the novel substrates of the present invention, under conditions which would allow measurable γ-secretase activity, through cleavage of the substrate, to occur. In this fashion, one can measure the ability of the candidate substance to stimulate the activity of the γ-secretase in the absence of the candidate substance. Likewise, in assays for inhibitors after obtaining a cell membrane fraction expressing functional γ-secretase, the candidate substance is admixed with that fraction. In this fashion the ability of the candidate inhibitory substance to reduce, abolish, or otherwise diminish a biological effect mediated by γ-secretase may be detected.

"Effective amounts" of the substance in certain circumstances are those amounts effective to reproducibly alter a given CT-100 cleaving γ-secretase activity or APP processing. These effective amounts are those which alter the degree or amount of cleavage of the APP CT-100 but do not alter the cleavage of the Notch fusion proteins of the present invention at γ-secretase cleavage site in comparison to the cleavage seen in the absence of the candidate substance. Compounds that will be particularly useful as therapeutic agents and/or for further characterization, are those compounds that preferentially inhibit APP cleavage by the γ-secretase as compared to Notch cleavage. By "preferentially inhibit" it is meant that the agent has more of an inhibitory effect on γ-secretase mediated cleavage of APP than on Notch. While it would be preferable that the agent is one which has no inhibitory effect on the Notch cleavage, some inhibition of the Notch cleavage may be acceptable so long as it is less than the APP cleavage seen by the wild-type γ-secretase enzyme.

The assays described above employing the novel γ-secretase substrates of the invention are amenable to numerous high throughput screening (HTS) methods (For a review see Jayawickreme and Kost, Curr. Opin. Biotechnol. 8:629-634, 1997). Automated and miniaturized HTS assays are also contemplated as described for example in Houston and Banks Cuir. Opin. Biotechnol. 8:734-740, 1997.

There are a number of different libraries used for the identification of small molecule modulators including chemical libraries, natural product libraries and combinatorial libraries comprised or random or designed peptides, oligonucleotides or organic molecules. Chemical libraries consist of structural analogs of known compounds or compounds that are identified as hits or leads via natural product screening or from screening against a potential therapeutic target. Natural product libraries are collections of products from microorganisms, animals, plants, insects or marine organisms which are used to create mixtures of screening by, e.g., fermentation and extractions of broths from soil, plant or marine organisms. Natural product libraries include polypeptides, non-nbosomal peptides and non-naturally occurring variants thereof. For a review see Science 282:63-68, 1998.

Combinatorial libraries are composed of large numbers of peptides oligonucleotides or organic compounds as a mixture. They are relatively simple to prepare by traditional automated synthesis methods, PCR cloning or other synthetic methods. Of particular interest will be libraries that include peptide, protein, peptidomimetic, multiparallel synthetic collection, recombinatorial and polypeptide libraries. A review of combinatorial libraries and libraries created therefrom, see Myers Curr. Opin. Biotechnol. 8:701-707, 1997. A modulator identified by the use of various libraries described may then be optimized to modulate activity of γ-secretase through, for example, rational drug design.

It will, of course, be understood that all the screening methods of the present invention are useful in themselves notwithstanding the fact that effective candidates may not be found. The invention provides methods for screening for such candidates, not solely methods of finding them

### C. In Vivo Assays

The present invention also encompasses the use of various animal models. Once the modulators have been screened in an *in vitro* environment as discussed above, any non-human models of APP processing and/or AD may be used to determine the in vivo effects of the modulators. This will afford an excellent opportunity to examine the function of γ-secretase in a whole animal system where it is normally expressed.

Treatment of animals with test compounds that have been identified as modulators of γ-secretase activity will involve the administration of the compound, in an appropriate form, to the animal. Administration will be by any route that can be utilized for clinical or non-clinical purposes, including but not limited to oral, nasal, buccal, rectal, vaginal or topical. Alternatively, administration may be by intratracheal instillation, bronchial instillation, intradermal, subcutaneous, intramuscular, intraperitoneal or intravenous injection. Specifically contemplated are systemic intravenous injection, regional administration via blood, cerebrospinal fluid (CSF) or lymph supply and intratumoral injection.

Determining the effectiveness of a compound *in vivo* may involve a variety of different criteria. Such criteria include, but are not limited to, survival, increased activity level, and improved food intake. Other methods of evaluation include pathological examination, especially of brain tissue, to look for indicia of altered γ-secretase activity, such as reduced production of amyloid beta or amyloid beta plaques and reduced atrophy of the brain.

### D. Manufacture of Medicaments

The assays of the invention will identify γ-secretase modulators that represent candidate therapeutics for treatment of diseases characterized by aberrant levels of γ-secretase activity, including AD. Thus, after identifying modulator agents, the methods of the invention optionally include the additional step or steps of manufacturing/synthesizing the agents, and of formulating the agent into a composition using pharmaceutically acceptable diluents, adjuvants, or carriers. Pharmaceutical compositions are described in greater detail below.

### VI. Pharmaceutical Compositions

The modulators of Notch processing APP processing, and/or γ-secretase cleavage identified by the present invention may ultimately be formulated into pharmaceutical compositions i.e., in a form appropriate for *in vivo* applications. Generally, this will entail preparing compositions that are essentially free of pyrogens, as well as other impurities that could be harmful to humans or animals.

One will generally desire to employ appropriate salts and buffers to render the identified modulator compositions stable and allow for their uptake by target cells. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the modulators identified by the present invention, its use in therapeutic compositions is contemplated. Supplementary active ingredients also can be incorporated into the compositions.

The modulator compositions of the present invention include classic pharmaceutical preparations. Administration of these compositions according to the present invention will be via any common route so long as the target tissue is available via that route. The pharmaceutical compositions may be introduced into the subject by any conventional method, e.g., by intravenous, intradermal, intramusclar, intramammary, intraperitoneal, intrathecal, intraocular, retrobulbar, intrapulmonary (e.g., term release); by oral, sublingual, nasal, anal, vaginal, or transdermal delivery, or by surgical implantation at a particular site, e.g., embedded under the splenic capsule, brain, or in the cornea. The treatment may consist of a single dose or a plurality of doses over a period of time.

The modulator compounds identified using the present invention may be prepared for administration as solutions of free base or pharmacologically acceptable salts in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated Supplementary active ingredients also can be incorporated into the compositions.

For oral administration the modulators identified by the present invention may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient may also be dispersed in dentifrices, including: gels, pastes, powders and slurries. The active ingredient may be added in a therapeutically effective amount to a paste dentifrice that may include water, binders, abrasives, flavoring agents, foaming agents, and humectants.

The compositions of the present invention may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like.

The compositions of the present invention may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration.

"Unit dose" is defined as a discrete amount of a therapeutic composition dispersed in a suitable carrier. For example, parenteral administration may be carried out with an initial bolus followed by continuous infusion to maintain therapeutic circulating levels of drug product. Those of ordinary skill in the art will readily optimize effective dosages and administration regimens as determined by good medical practice and the clinical condition of the individual patient. More particularly, the dose should be selected to reduce, inhibit, decrease or otherwise abrogate the formation of Aβ-peptide and more particularly, plaque formation in the brain of a subject exhibiting AD. To this effect, those of skill in the art will be able to employ animal models of AD (e.g., as disclosed in U.S. Patent No.5,877,399; U.S. Patent No. 5,387,742; U.S. Patent No 5,811,633) in order to optimize dose administration protocols and predict the relevant amounts of pharmaceutical agents required for intervention of AD in a human subject.

The frequency of dosing will depend on the pharmacokinetic parameters of the agents and the routes of administration. The optimal pharmaceutical formulation will be determined by one of skill in the art depending on the route of administration and the desired dosage. See for example Remington's Pharmaceutical Sciences, 18th Ed. (1990, Mack Publ. Co, Easton PA 18042) pp 1435-1712, incorporated herein by reference. Such formulations may influence the physical state, stability, rate of in vivo release and rate of in vivo clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein as well as the pharmacokinetic data observed in animals or human clinical trials.

Appropriate dosages may be ascertained through the use of established assays for determining blood levels in conjunction with relevant dose-response data. The final dosage regimen will be determined by the attending physician, considering factors which modify the action of drugs, e.g., the drug's specific activity, severity of the damage and the responsiveness of the patient, the age, condition, body weight, sex and diet of the patient, the severity of any infection, time of administration and other clinical factors. As studies are conducted, further information will emerge regarding appropriate dosage levels and duration of treatment for specific diseases and conditions.

It will be appreciated that the pharmaceutical compositions and treatment methods of the invention may be useful in fields of human medicine and veterinary medicine. Thus the subject to be treated may be a mammal, preferably human or other animal. For veterinary purposes, subjects include for example, farm animals including cows, sheep, pigs, horses and goats, companion animals such as dogs and cats, exotic and/or zoo animals, laboratory animals including mice rats, rabbits, guinea pigs and hamsters; and poultry such as chickens, turkey, ducks and geese.

### VI. Examples

The following examples are included to demonstrate preferred embodiments of the invention. However, those of skill in the art should, in light of the present disclosure, appreciate that many changes can be made in the specific embodiments which are disclosed and still obtain a like or similar result without departing from the spirit and scope of the invention.

### Example 1

### Materials and Methods

The present Example provides a teaching of the general techniques, reagents and assays employed to obtain the results discussed herein. General laboratory chemicals were purchased from Sigma Chemical Co (St. Louis, Mo). The pET 43.1a vector was from Novagen (Madison, WI) and restriction enzymes were from Invitrogen (Carlsbad, Ca). Oligonucleotides were from Sigma Genosys (The Woodlands, Tx). The Val-1744 antibody was from Cell Signaling Technology (Beverly, Ma). PHA/PNU compounds were obtained from the Pharmacia compound collection (New Jersey, USA).

**Cloning Notch Substrate:** Notch substrate containing amino acids N1703-D1860 of mouse notch-1 protein (DNA Sequence accession number Z11886) was cloned into pET 43.1 a vector as an EcoRI/HindIII insert in frame with a nucleic acid that encoded the NusA. The expression construct for encoding the NusA/Notch fusion protein had a sequence of SEQ ID NO: SEQ ID NO: 1. This construct contained C-terminal Flag and 8His tags generated through PCR to give the extension (DYKDDDDKHHHHHHHH, SEQ ID NO: 15) following amino acid 1860 of Notch. The DNA was transformed into BL21 (DE3) competent *E. coli* (Stratagene) and clones were screened for the presence of the correct insert using DNA Concert miniprep kit (Gibco/BRL). Clone Notch-F6 was found to contain the correct DNA sequence of SEQ ID NO1.

**Expression and Purification of Notch/NusA fusion protein.** E. coli was transformed with clone Notch-F6 was inoculated into LB/Amp and grown overnight at 37°C in a shaking incubator. The next day, 35 ml of the overnight culture was used to inoculate 2 liters of LB/Amp. The *E*. *coli* were grown at 37°C in a shaking incubator until the A₆₀₀ reached 0.4 and then were induced with 1 mM IPTG for 3 hours and centrifuged. Pellets from two liters of culture were resuspended in 5 ml/g pellet of 50 mM Tris, pH 8.0, 100mM NaCl including protease inhibitors and were processed three times with a French Press to yield a crude extract. The pH of the extract was adjusted to 8.0 using 2M Tris and was centrifuged at 11,000g for 45 min. The supernatant was loaded onto a 4 ml nickel IMAC chromatography column equilibrated in 50 mM Tris, pH 8.0, 100 mM NaCl, and with protease inhibitors. Column was washed with the same buffer followed by buffer containing 50 mM imidazole. NusA-Notch fusion protein was eluted with buffer containing 300 mM imidazole and 0.8 ml fractions were collected and analyzed by A₂₈₀ and SDS-PAGE. Fractions containing NusA-Notch were pooled and dialyzed into 50 mM Pipes, pH 7.0, 100 mM NaCl.

**Cleavage of Notch as Determined by Western Blot Analysis.** NusA-Notch fusion (1.7 µM) was incubated with 70 µg/ml solubilized γ-secretase in 50 mM Pipes, pH 7.0, 0.25% CHAPSO in a total volume of 50 µl overnight at 37°C. DAPT (PHA-568638) was added at varying concentrations. The reactions were stopped with the addition of 12.5 µl of 5X Laemmli buffer (Laemmli 1970) and 30 µl of the mixture was electrophoresed on a 15% SDS-PAGE. Proteins were transferred to nitrocellulose using a semi-dry blot apparatus (Millipore) and blocked for 2 hours using 4% BSA in PBS/0.5% Tween-20. Val-1744 antibody was added to the blocking solution at a 1:1000 dilution and incubated for 1 hour. The membrane was washed three times with PBS/0.5% Tween-20 followed by incubation with anti-rabbit IgG-HRP (1:5000 dil in 4% BSA in PBS/0.5% Tween-20). The membrane was again washed three times and developed using ECL reagents (Amersham, Piscataway, New Jersey).

***In vitro Notch Cleavage ELISA.*** Notch cleavage was also assessed using an ELISA technique. Prior to setting up a reaction, the required number of wells in a 96-well half-area plate (Costar) were coated with 50 µl ofVal-1744 antibody diluted 1:200 in 0.1M NaHCO₃, pH 8.2. Plates were incubated overnight at 4°C. The Notch cleavage reaction was set up as follows. NusA-Notch (0.9 µM) was incubated with 70 µg/ml of solubilized γ-secretase in 50 mM Pipes, pH 7.0, 0.25% CHAPSO in a total volume of 25 µl overnight at 37°C. The next day, the plate coated with Val-1744 antibody was washed 3 times with PBS/0.05% Tween-20 and blocked using 4% BSA in PBS/0.05% Tween-20 for 1 hour. The cleavage reaction mix was diluted 14-fold using 4% BSA in PBS/0.05% Tween-20 and 50 µl was plated in triplicate and incubated for 3-4 hours at room temp. Plates were washed three times with PBS/0.05% Tween-20 and 50 µl anti-FLAG-HRP antibody (Sigma, St. Louis, Mo) used at 1:60000 dilution in 4% BSA/PBS/0.5% Tween-20 was added. This antibody was incubated for 45 min and the plate washed three times with PBS/0.5% Tween-20. TMB reagent (Kirkegaard & Perry) was mixed 1:1 and 50 µl was added to the wells. The color was allowed to develop for 1 hour and 50 µl of 1M H₃PO₄ was added and the plates read at 450 nm on a SpectraMax Plus plate reader. When varying the Notch substrate concentration, 0.11 to 3.6 µM of NusA-Notch was used. When the enzyme concentration was varied, 2.1 to 68 µg/ml solubilized γ-secretase was used.

**Inhibition of Notch Cleavage.** Inhibitors (1 µl) were added to the cleavage reaction as 25x concentrations in 50% DMSO prior to the addition of the enzyme. Blanks and no inhibitor controls were adjusted to contain the same final DMSO concentration. IC₅₀'s were calculated for inhibitors using the 4-parameter logistic model in the GraFit 4.0 program.

### Example 2

### Results and Discussion

In order to produce a soluble Notch substrate, the inventors prepared a number of constructs in *E. coli* (Figure 3a). These include the use of caspase leader, ubiquitin, N-terminal tau, and the Nus tag. Soluble expression was observed only with Nus fusion (Wilkinson et al., Bio/Technology 9:443-448, 1991). Figure 3b shows cloning details for this construct. To facilitate purification and assay development, a His and a Flag tag were engineered to the C-terminus of Notch.

When the Nus-tagged Notch fusion protein was expressed in *E. coli,* a high level expression of the fusion protein corresponding to a 90 kDa band was observed on a SDS-PAGB (Figure 4, Lane 1), a size expected from the fusion protein. When the total lysate was centrifuged, the fusion protein remained in the soluble fraction (Figure 4, lane 2). Notch-containing fusion protein was purified from the soluble fraction by IMAC using nickel as the immobilized metal ion. Figure 4 (lanes 5-9) shows various fractions eluted from the IMAC column by 300 mM imidazole. These fractions were pooled, dialyzed, and then used as a source of the substrate for γ-secretase cleavage.

The technique for following a specific cleavage in the Notch protein is based on the specificity of Val-1744 antibody (Cell Signaling Technology). It is specific for cleaved Notch and does not cross react with uncleaved Notch protein. As shown in Figure 5 (lane 3), the cleaved Notch protein was detected on a Western blot with antibody that is specific for Val-1744. As shown in lane 1, no cross reactivity to the uncleaved Notch fusion protein was observed. Moreover, this specific cleavage in Notch protein was inhibited in a dose-dependent manner by DAPT (Dovey et al., J. Neurochem. 76:173-181, 2001), a well-known potent inhibitor of γ-secretase (lanes 4-8, Figure 5).

In order to determine if there is an additional cleavage in the Notch protein, the cleavage reaction was also monitored by a Western blot using the C-terminal Flag antibody. Out of the three immunoreactive bands, only one cleavage product was inhibitable by DAPT. Taken together, these results suggest that *in vitro,* γ-secretase-mediated cleavage seems to result in a specific cleavage at the 1743/1744, consistent with cell based studies showing the production of NICD from Notch (Kopan et al., Proc. Natl. Acad. Sci. 93:1683-1688, 1996; Schroeter et al., Nature, 393:382-386, 1998).

The above results showed that Notch fusion protein is susceptible to a γ-secretase-mediated specific cleavage which can be detected on a Western blot by a highly specific Val-1744 monoclonal antibody. Since Western blots are not quantitative, it is difficult to evaluate compounds for Notch inhibition and compare the inhibitory potencies to the Aβ ELISA.

Figure 6 shows a strategy for the development of a quantitative ELISA for Notch cleavage for comparison of inhibitory potency of compounds with the CT-100-based ELISA. It is based on a sandwich ELISA using the highly specific anti-Val 1744 and anti Flag antibodies to capture the N- and C-terminus of the NICD fragment from the Notch fusion protein in the presence of γ-secretase. This assay is performed as described in Example 1 under the heading *"In vitro* Notch Cleavage ELISA". Essentially, in this sandwich ELISA, the microtiter plate is coated Val-1744 antibody. Meanwhile the NusA-Notch cleavage reaction is performed in which NusA-Notch is incubated with γ-secretase. The plate coated with Val-1744 antibody is washed with buffer and blocked with BSA. The cleavage reaction mixture is then added to the microtiter plate and incubated for an appropriate time period at room temp. Plates are washed anti-FLAG-HRP antibody (Sigma, St. Louis, Mo) is added and again incubated for an appropriate time. The chromogenic substrate for HRP, TMB reagent (Kirkegaard & Perry) is added and the color once developed is read at 450 nm on a SpectraMax Plus plate reader. Figure 7 shows cleavage of NusA-Notch fusion protein as detected by ELISA. A 5-fold signal to noise ratio was observed in the ELISA

The enzymatic activity of solubilized γ-secretase for Notch cleavage was further characterized in the ELISA under defined experimental conditions. Figure 8A shows the substrate dependence on product formation. The reaction obeys a Michaelis-Menten kinetics and the apparent Kₘ for hydrolysis of Notch fusion protein substrate by solubilized γ-secretase activity is about 0.7 µM under defined conditions. The observed Notch cleavage activity was also linear with protein concentration of solubilized membrane preparation containing γ-secretase activity (Figure 8B).

Further confirmation of γ-secretase-mediated Notch cleavage in the ELISA was obtained by inhibition studies using specific inhibitors of this enzyme reported in the literature. A potent inhibitor of γ-secretase, called DAPT, has been reported (Dovey et al., J. Neurochem. 76:173-181, 2001). As show in Figure 9, DAPT (PHA-568638) inhibits Notch cleavage in a dose-dependent manner with an IC₅₀ = 2.4 nM. On the other hand, fenchylamine sulfonamide (PHA-512088) has been reported (Rishton et al., J. Med. Chem. 43:2297-2299, 2000) to inhibit γ-secretase activity in the low µM range in the CT-100 *in vitro* assay. As shown in Figure 9, PHA-512088 inhibited γ-secretase-mediated Notch cleavage with an IC₅₀ of 0.7 µM in the ELISA under defined conditions. As shown, inhibitors (PHA-568638; PHA-512088) in the low nM to low µM range work very well in the assay. Taken together, these results show that *in vitro* enzymatic activity producing a NICD fragment from a Notch fusion protein substrate is due to γ-secretase.

Recently, cell based assays to follow APP and Notch cleavage in parallel have been reported (Karlstrom et al., J. Biol. Chem. 277-6763-6766,2002). However, the present invention for the first time demonstration an *in vitro* quantitative ELISA for following specific Notch cleavage (G1743-V1744) by γ-secretase. The present invention for the first time demonstrates that γ-secretase activity from HeLa cells specifically cleaves the Notch protein at the 1743-1744 junction.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention More specifically, it will be apparent that certain agents which are both chemically and physiologically related may be substituted for the agents described herein while the same or similar results would be achieved. All such similar substitutes and modifications apparent to those skilled in the art are deemed to be within the scope of the invention as defined by the appended claims.

## Claims

1. A soluble fusion protein comprising recombinant Notch protein fused to the C-terminus of a NusA protein sequence. '

2. The soluble fusion protein of claim 1, wherein said recombinant Notch protein comprises the S3 cleavage site of Notch.

3. The soluble fusion protein of claim 1, wherein said recombinant Notch protein is a vertebrate Notch protein.

4. The soluble fusion protein of claim 1, wherein said recombinant Notch protein is an invertebrate Notch protein.

5. The soluble fusion protein of claim 1, wherein said recombinant Notch protein is derived from mouse Notch protein having the sequence of SEQ ID NO:5.

6. The soluble fusion protein of claim 1, wherein said recombinant Notch protein comprises amino acids 1703 through 1860 of mouse Notch protein. Additional claims to be added to Druckexemplar

7. The soluble fusion protein of claim 6, wherein said recombinant Notch protein comprises amino acids 1703 through 1860 of the mouse Notch protein as set forth in SEQ ID NO: 5, and wherein said NusA protein sequence comprises SEQ ID NO: 17.

8. The soluble fusion protein of claim 7, wherein said recombinant Notch protein consists essentially of amino acids 1703 through 1860 of the mouse Notch protein as set forth in SEQ ID NO: 5, and wherein said NusA protein sequence consists essentially of SEQ ID NO: 17.

9. The soluble fusion protein of claim 8, encoded by the nucleotide sequence as set forth in SEQ ID NO: 1.

10. The soluble fusion protein of any of claims 1 through 6, further comprising a C-terminal His-tag.

11. The soluble fusion protein of any of claims 1 through 6, further comprising a C-terminal Flag-tag.

12. A polynucleotide comprising a nucleotide sequence that encodes a fusion protein according to any one of claims 1 to 11.

13. A polynucleotide sequence that encodes a fusion protein of claim 1, wherein said polynucleotide sequence comprises a sequence set forth in SEQ ID NO: 1.

14. An *in vitro* method of assaying for γ-secretase mediated ε cleavage (1743/1744) of Notch protein comprising:
a. contacting a first composition comprising a mammalian γ-secretase complex or biologically active fragment thereof, with a second composition comprising a fusion protein according to any of claims 1 through 11 and
b. measuring cleavage of the fusion protein.

15. An *in vitro* method of screening for modulators of γ-secretase mediated ε cleavage (1743/1744) of Notch protein, comprising the steps of
(a) contacting a first composition comprising a mammalian γ-secretase complex or biologically active fragment thereof, with a second composition comprising a fusion protein according to any of claims 1 through 11 in the presence and in the absence of a putative modulator compound; and
(b) measuring cleavage of the fusion protein in the presence and in the absence of a putative modulator compound; and
(c) identifying modulators which modulate the γ-secretase mediated cleavage of said fusion protein;
wherein a putative modulator compound produces a difference in γ-secretase cleavage in step (b).

16. A kit for performing a γ-secretase assay comprising a γ-secretase substrate comprising a fusion protein according to any of claims 1 through 11.

17. A fusion protein comprising a NusA polypeptide fused to a Notch polypeptide comprising between 90 to 95% sequence identity with a NusA sequence of SEQ ID NO:17, wherein the Notch polypeptide comprises the transmembrane domain of Notch, and wherein the fusion protein is soluble in an aqueous solution.

18. A method for screening for a selective inhibitor of γ-secretase processing of amyloid precursor protein (APP), comprising:
a) providing a test compound which inhibits γ-secretase mediated cleavage of a polypeptide comprising an APP gamma secretase site; and
b) measuring gamma secretase cleavage of a fusion protein according to claim 1 in the presence and absence of the test compound;
wherein a test compound that preferentially inhibits gamma secretase cleavage of said polypeptide compared to cleavage of said fusion protein is a selective inhibitor of gamma secretase processing of APP.

## Patentansprüche

1. Lösliches Fusionsprotein, umfassend ein rekombinantes Notch-Protein, fusioniert an den C-Terminus einer NusA-Proteinsequenz.

2. Lösliches Fusionsprotein nach Anspruch 1, wobei das rekombinante Notch-Protein die S3-Spaltstelle von Notch umfaßt.

3. Lösliches Fusionsprotein nach Anspruch 1, wobei das rekombinante Notch-Protein ein vertebrales Notch-Protein ist.

4. Lösliches Fusionsprotein nach Anspruch 1, wobei das rekombinante Notch-Protein ein invertebrales Notch-Protein ist.

5. Lösliches Fusionsprotein nach Anspruch 1, wobei das rekombinante Notch-Protein aus dem Maus-Notch-Protein mit der Sequenz von SEQ ID NO: 5 stammt.

6. Lösliches Fusionsprotein nach Anspruch 1, wobei das rekombinante Notch-Protein die Aminosäuren 1703 bis 1860 des Maus-Notch-Proteins umfaßt.

7. Lösliches Fusionsprotein nach Anspruch 6, wobei das rekombinante Notch-Protein die Aminosäuren 1703 bis 1860 des Maus-Notch-Proteins, wie in SEQ ID NO: 5 angegeben, umfaßt, und wobei die NusA-Proteinsequenz SEQ ID NO: 17 umfaßt.

8. Lösliches Fusionsprotein nach Anspruch 7, wobei das rekombinante Notch-Protein im wesentlichen aus den Aminosäuren 1703 bis 1860 des Maus-Notch-Proteins, wie in SEQ ID NO: 5 angegeben, besteht, und wobei die NusA-Proteinsequenz im wesentlichen aus SEQ ID NO: 17 besteht.

9. Lösliches Fusionsprotein nach Anspruch 8, kodiert durch die Nukleotidsequenz, angegeben durch SEQ ID NO: 1.

10. Lösliches Fusionsprotein nach einem der Ansprüche 1 bis 6, ferner umfassend eine C-terminale His-Markierung.

11. Lösliches Fusionsprotein nach einem der Ansprüche 1 bis 6, ferner umfassend eine C-terminale Flag-Markierung.

12. Polynukleotid, umfassend eine Nukleotidsequenz, die ein Fusionsprotein nach einem der Ansprüche 1 bis 11 kodiert.

13. Polynukleotidsequenz, die ein Fusionsprotein nach Anspruch 1 kodiert, wobei die Polynukleotidsequenz eine Sequenz, angegeben in SEQ ID NO: 1, umfaßt.

14. In-vitro-Verfahren zum Test auf die γ-Secretase-vermittelte ε-Spaltung (1743/1744) des Notch-Proteins, umfassend:
a. Kontaktieren einer ersten Zusammensetzung, umfassend einen Säuger-γ-Secretasekomplex oder ein biologisch aktives Fragment davon, mit einer zweiten Zusammensetzung, umfassend ein Fusionsprotein nach einem der Ansprüche 1 bis 11; und
b. Messen der Spaltung des Fusionsproteins.

15. In-vitro-Verfahren zum Screenen auf Modulatoren der γ-Secretase-vermittelten ε-Spaltung (1743/1744) des Notch-Proteins, umfassend die folgenden Schritte:
(a) Kontaktieren einer ersten Zusammensetzung, umfassend einen Säuger-γ-Secretasekomplex oder ein biologisch aktives Fragment davon, mit einer zweiten Zusammensetzung, umfassend ein Fusionsprotein nach einem der Ansprüche 1 bis 11, in Gegenwart und in Abwesenheit einer putativen Modulatorverbindung;
(b) Messen der Spaltung des Fusionsproteins in Gegenwart und Abwesenheit einer mutmaßlichen Modulatorverbindung und
(c) Identifizieren von Modulatoren, die die γ-Secretase-vermittelte Spaltung des Fusionsproteins modulieren; wobei eine mutmaßliche Modulatorverbindung einen Unterschied bei der γ-Secretasespaltung in Schritt (b) erzeugt.

16. Kit zur Durchführung eines γ-Secretaseassays, umfassend ein γ-Secretasesubstrat, umfassend ein Fusionsprotein nach einem der Ansprüche 1 bis 11.

17. Fusionsprotein, umfassend ein an ein Notch-Polypeptid fusioniertes NusA-Polypeptid, umfassend 90 bis 95 % Sequenzidentität mit der NusA-Sequenz von SEQ ID NO: 17, wobei das Notch-Polypeptid die Transmembrandomäne von Notch umfaßt und wobei das Fusionsprotein in einer wässerigen Lösung löslich ist.

18. Verfahren zum Screenen auf einen selektiven Inhibitor der γ-Secretaseprozessierung eines Amyloidpräkursorproteins (APP), umfassend:
a) Bereitstellen einer Testverbindung, die die γ-Secretase-vermittelte Spaltung eines Polypeptids, umfassend eine APP-gamma-Secretasestelle, inhibiert; und
b) Messen der gamma-Secretasespaltung eines Fusionsproteins nach Anspruch 1 in Gegenwart und Abwesenheit der Testverbindung,
wobei eine Testverbindung, die bevorzugt die gamma-Secretasespaltung des Polypeptids im Vergleich zu der Spaltung des Fusionsproteins inhibiert, ein selektiver Inhibitor der gamma-Secretaseprozessierung von APP ist.

## Revendications

1. Protéine de fusion soluble comprenant une protéine Notch recombinante fusionnée au C-terminal d'une séquence protéinique NusA.

2. Protéine de fusion soluble selon la revendication 1, dans laquelle ladite protéine Notch recombinante comprend le site de clivage S3 de Notch.

3. Protéine de fusion soluble selon la revendication 1, dans laquelle ladite protéine Notch recombinante est une protéine Notch de vertébré.

4. Protéine de fusion soluble selon la revendication 1, dans laquelle ladite protéine Notch recombinante est une protéine Notch d'invertébré.

5. Protéine de fusion soluble selon la revendication 1, dans laquelle ladite protéine Notch recombinante dérive d'une protéine Notch de souris ayant la séquence SEQ ID NO:5.

6. Protéine de fusion soluble selon la revendication 1, dans laquelle ladite protéine Notch recombinante comprend les acides aminés 1703 à 1860 d'une protéine Notch de souris.

7. Protéine de fusion soluble selon la revendication 6, dans laquelle ladite protéine Notch recombinante comprend les acides aminés 1703 à 1860 de la protéine Notch de souris comme énoncé dans SEQ ID NO:5 et dans laquelle ladite séquence protéinique NusA comprend SEQ ID NO:17.

8. Protéine de fusion soluble selon la revendication 7, dans laquelle ladite protéine Notch recombinante consiste essentiellement en les acides aminés 1703 à 1860 de la protéine Notch de souris comme énoncé dans SEQ ID NO:5 et dans laquelle ladite séquence protéinique NusA consiste essentiellement en SEQ ID NO:17.

9. Protéine de fusion soluble selon la revendication 8, codée par la séquence nucléotidique énoncée dans SEQ ID NO:1.

10. Protéine de fusion soluble selon l'une quelconque des revendications 1 à 6, comprenant en outre une étiquette His en C-terminal

11. Protéine de fusion soluble selon l'une quelconque des revendications 1 à 6, comprenant en outre une étiquette Flag en C-terminal.

12. Polynucléotide comprenant une séquence nucléotidique qui code une protéine de fusion selon l'une quelconque des revendications 1 à 11.

13. Séquence polynucléotidique qui code une protéine de fusion selon la revendication 1, ladite séquence polynucléotidique comprenant une séquence telle qu'énoncé dans SEQ ID NO:1.

14. Procédé de test in vitro du ε-clivage (1743/1744) de la protéine Notch médié par une γ-secrétase, comprenant :
a. la mise en contact d'une première composition comprenant un complexe γ-secrétase de mammifère ou un fragment biologiquement actif correspondant, avec une seconde composition comprenant une protéine de fusion selon l'une quelconque des revendications 1 à 11 ; et
b. la mesure du clivage de la protéine de fusion.

15. Procédé de criblage in vitro de modulateurs de ε-clivage (1743/1744) de la protéine Notch médié par une γ-secrétase, comprenant les étapes de :
a. mise en contact d'une première composition comprenant un complexe γ-secrétase de mammifère ou un fragment biologiquement actif correspondant, avec une seconde composition comprenant une protéine de fusion selon l'une quelconque des revendications 1 à 11, en présence et en l'absence d'un composé modulateur putatif ;
b. mesure du clivage de la protéine de fusion en présence et en l'absence d'un composé modulateur putatif ;
c. identification de modulateurs qui modulent le clivage de ladite protéine de fusion médié par une γ-secrétase ;
procédé dans lequel un composé modulateur putatif produit une différence dans l'étape du clivage, par la γ-secrétase, dans l'étape (b).

16. Kit pour mettre en oeuvre un test de γ-secrétase comprenant un substrat pour γ-secrétase comprenant une protéine de fusion selon l'une quelconque des revendications 1 à 11.

17. Protéine de fusion comprenant un polypeptide NusA fusionné à un polypeptide Notch comprenant entre 90 et 95% d'identité de séquence avec une séquence NusA de SEQ ID NO:17, le polypeptide Notch comprenant le domaine transmembranaire de Notch et la protéine de fusion étant soluble dans une solution aqueuse.

18. Procédé de criblage d'un inhibiteur sélectif de la transformation par une γ-secrétase d'une protéine précurseur amyloïde (APP), comprenant :
a. la fourniture d'un composé à tester qui inhibe le clivage d'un polypeptide comportant un site APP gamma secrétase, clivage médié par la γ-secrétase, et
b. la mesure du clivage gamma secrétase d'une protéine de fusion selon la revendication 1 en présence et en l'absence du composé testé ;
procédé dans lequel un composé testé qui inhibe préférentiellement le clivage dudit polypeptide par la gamma secrétase par comparaison au clivage de ladite protéine de fusion est un inhibiteur sélectif de la transformation de l'APP par une γ-secrétase.


### SEQUENCE LISTING

<110> Sharma et al.
<120> SOLUBLE NOTCH-BASED SUBSTRATES FOR GAMMA SECRETASE AND METHODS AND COMPOSITIONS FOR USING SAME
<130> 28341/01130
<160> 17
<170> PatentIn version 3.1
<210> 1
   <211> 2190
   <212> DNA
   <213> Artificial sequence
<220>
   <223> DNA encoding synthetic fusion of notch and nus
<400> 1
   atgaacaaag aaattttggc tgtagttgaa gccgtatcca atgaaaaggc gctacctcgc    60
   gagaagattt tcgaagcatt ggaaagcgcg ctggcgacag caacaaagaa aaaatatgaa   120
   caagagatcg acgtccgcgt acagatcgat cgcaaaagcg gtgattttga cactttccgt   180
   cgctggttag ttgttgatga agtcacccag ccgaccaagg aaatcaccct tgaagccgca   240
   cgttatgaag atgaaagcct gaacctgggc gattacgttg aagatcagat tgagtctgtt   300
   acctttgacc gtatcactac ccagacggca aaacaggtta tcgtgcagaa agtgcgtgaa   360
   gccgaacgtg cgatggtggt tgatcagttc cgtgaacacg aaggtgaaat catcaccggc   420
   gtggtgaaaa aagtaaaccg cgacaacatc tctctggatc tgggcaacaa cgctgaagcc   480
   gtgatcctgc gcgaagatat gctgccgcgt gaaaacttcc gccctggcga ccgcgttcgt   540
   ggcgtgctct attccgttcg cccggaagcg cgtggcgcgc aactgttcgt cactcgttcc   600
   aagccggaaa tgctgatcga actgttccgt attgaagtgc cagaaatcgg cgaagaagtg   660
   attgaaatta aagcagcggc tcgcgatccg ggttctcgtg cgaaaatcgc ggtgaaaacc   720
   aacgataaac gtatcgatcc ggtaggtgct tgcgtaggta tgcgtggcgc gcgtgttcag   780
   gcggtgtcta ctgaactggg tggcgagcgt atcgatatcg tcctgtggga tgataacccg   840
   gcgcagttcg tgattaacgc aatggcaccg gcagacgttg cttctatcgt ggtggatgaa   900
   gataaacaca ccatggacat cgccgttgaa gccggtaatc tggcgcaggc gattggccgt   960
   aacggtcaga acgtgcgtct ggcttcgcaa ctgagcggtt gggaactcaa cgtgatgacc  1020
   gttgacgacc tgcaagctaa gcatcaggcg gaagcgcacg cagcgatcga caccttcacc  1080
   aaatatctcg acatcgacga agacttcgcg actgttctgg tagaagaagg cttctcgacg  1140
   ctggaagaat tggcctatgt gccgatgaaa gagctgttgg aaatcgaagg ccttgatgag  1200
   ccgaccgttg aagcactgcg cgagcgtgct aaaaatgcac tggccaccat tgcacaggcc  1260
   caggaagaaa gcctcggtga taacaaaccg gctgacgatc tgctgaacct tgaaggggta  1320
   gatcgtgatt tggcattcaa actggccgcc cgtggcgttt gtacgctgga agatctcgcc  1380
   gaacagggca ttgatgatct ggctgatatc gaagggttga ccgacgaaaa agccggagca  1440
   ctgattatgg ctgcccgtaa tatttgctgg ttcggtgacg aagcgactag tggttctggt  1500
   catcaccatc accatcactc cgcgggtaaa gaaaccgctg ctgcgaaatt tgaacgccag  1560
   cacatggact cgccaccgcc aactggtctg gtcccccggg gcagcgcggg ttctggtacg  1620
   attgatgacg acgacaagag tccgggagct cgtggatccg aattcaatat tccttacaag  1680
   attgaggccg tgaagagtga gccggtggag cctccgctgc cctcgcagct gcacctcatg  1740
   tacgtggcag cggccgcctt cgtgctcctg ttctttgtgg gctgtggggt gctgctgtcc  1800
   cgcaagcgcc ggcggcagca tggccagctc tggttccctg agggtttcaa agtgtcagag  1860
   gccagcaaga agaagcggag agagcccctc ggcgaggact cagtcggcct caagcccctg  1920
   aagaatgcct cagatggtgc tctgatggac gacaatcaga acgagtgggg agacgaagac  1980
   ctggagacca agaagttccg gtttgaggag ccagtagttc tccctgacct gagtgatcag  2040
   actgaccaca gacagtggac ccagcagcac ctggacgctg ctgacctgcg catgtctgcc  2100
   atggccccaa caccgcctca gggggaggtg gatgctgacg attataaaga cgatgacgat  2160
   aaacaccatc accatcacca tcaccattga                                    2190
<210> 2
   <211> 729
   <212> PRT
   <213> Artificial sequence
<220>
   <223> Synthetic fusion protein sequence of notch and nus
<400> 2
   Met Asn Lys Glu Ile Leu Ala Val Val Glu Ala Val Ser Asn Glu Lys
   1               5                   10                  15
   Ala Leu Pro Arg Glu Lys Ile Phe Glu Ala Leu Glu Ser Ala Leu Ala
               20                  25                  30
   Thr Ala Thr Lys Lys Lys Tyr Glu Gln Glu Ile Asp Val Arg Val Gln
           35                  40                  45
   Ile Asp Arg Lys Ser Gly Asp Phe Asp Thr Phe Arg Arg Trp Leu Val
       50                  55                  60
   Val Asp Glu Val Thr Gln Pro Thr Lys Glu Ile Thr Leu Glu Ala Ala
   65                  70                  75                  80
   Arg Tyr Glu Asp Glu Ser Leu Asn Leu Gly Asp Tyr Val Glu Asp Gln
                   85                  90                  95
   Ile Glu Ser Val Thr Phe Asp Arg Ile Thr Thr Gln Thr Ala Lys Gln
               100                 105                 110
   Val Ile Val Gln Lys Val Arg Glu Ala Glu Arg Ala Met Val Val Asp
           115                 120                 125
   Gln Phe Arg Glu His Glu Gly Glu Ile Ile Thr Gly Val Val Lys Lys
       130                 135                 140
   Val Asn Arg Asp Asn Ile Ser Leu Asp Leu Gly Asn Asn Ala Glu Ala
   145                 150                 155                 160
   Val Ile Leu Arg Glu Asp Met Leu Pro Arg Glu Asn Phe Arg Pro Gly
                   165                 170                 175
   Asp Arg Val Arg Gly Val Leu Tyr Ser Val Arg Pro Glu Ala Arg Gly
               180                 185                 190
   Ala Gln Leu Phe Val Thr Arg Ser Lys Pro Glu Met Leu Ile Glu Leu
           195                 200                 205
   Phe Arg Ile Glu Val Pro Glu Ile Gly Glu Glu Val Ile Glu Ile Lys
       210                 215                 220
   Ala Ala Ala Arg Asp Pro Gly Ser Arg Ala Lys Ile Ala Val Lys Thr
   225 230 235 240
   Asn Asp Lys Arg Ile Asp Pro Val Gly Ala Cys Val Gly Met Arg Gly
   245 250 255
   Ala Arg Val Gln Ala Val Ser Thr Glu Leu Gly Gly Glu Arg Ile Asp
   260 265 270
   Ile Val Leu Trp Asp Asp Asn Pro Ala Gln Phe Val Ile Asn Ala Met
   275 280 285
   Ala Pro Ala Asp Val Ala Ser Ile Val Val Asp Glu Asp Lys His Thr
   290 295 300
   Met Asp Ile Ala Val Glu Ala Gly Asn Leu Ala Gln Ala Ile Gly Arg
   305 310 315 320
   Asn Gly Gln Asn Val Arg Leu Ala Ser Gln Leu Ser Gly Trp Glu Leu
   325 330 335
   Asn Val Met Thr Val Asp Asp Leu Gln Ala Lys His Gln Ala Glu Ala
   340 345 350
   His Ala Ala Ile Asp Thr Phe Thr Lys Tyr Leu Asp Ile Asp Glu Asp
   355 360 365
   Phe Ala Thr Val Leu Val Glu Glu Gly Phe Ser Thr Leu Glu Glu Leu
   370 375 380
   Ala Tyr Val Pro Met Lys Glu Leu Leu Glu Ile Glu Gly Leu Asp Glu
   385 390 395 400
   Pro Thr Val Glu Ala Leu Arg Glu Arg Ala Lys Asn Ala Leu Ala Thr
   405 410 415
   Ile Ala Gln Ala Gln Glu Glu Ser Leu Gly Asp Asn Lys Pro Ala Asp
   420 425 430
   Asp Leu Leu Asn Leu Glu Gly Val Asp Arg Asp Leu Ala Phe Lys Leu
   435 440 445
   Ala Ala Arg Gly Val Cys Thr Leu Glu Asp Leu Ala Glu Gln Gly Ile
   450 455 460
   Asp Asp Leu Ala Asp Ile Glu Gly Leu Thr Asp Glu Lys Ala Gly Ala
   465 470 475 480
   Leu Ile Met Ala Ala Arg Asn Ile Cys Trp Phe Gly Asp Glu Ala Thr
   485 490 495
   Ser Gly Ser Gly His His His His His His Ser Ala Gly Lys Glu Thr
   500 505 510
   Ala Ala Ala Lys Phe Glu Arg Gln His Met Asp Ser Pro Pro Pro Thr
   515 520 525
   Gly Leu Val Pro Arg Gly Ser Ala Gly Ser Gly Thr Ile Asp Asp Asp
   530 535 540
   Asp Lys Ser Pro Gly Ala Arg Gly Ser Glu Phe Asn Ile Pro Tyr Lys
   545 550 555 560
   Ile Glu Ala Val Lys Ser Glu Pro Val Glu Pro Pro Leu Pro Ser Gln
   565 570 575
   Leu His Leu Met Tyr Val Ala Ala Ala Ala Phe Val Leu Leu Phe Phe
   580 585 590
   Val Gly Cys Gly Val Leu Leu Ser Arg Lys Arg Arg Arg Gln His Gly
   595 600 605
   Gln Leu Trp Phe Pro Glu Gly Phe Lys Val Ser Glu Ala Ser Lys Lys
   610 615 620
   Lys Arg Arg Glu Pro Leu Gly Glu Asp Ser Val Gly Leu Lys Pro Leu
   625 630 635 640
   Lys Asn Ala Ser Asp Gly Ala Leu Met Asp Asp Asn Gln Asn Glu Trp
   645 650 655
   Gly Asp Glu Asp Leu Glu Thr Lys Lys Phe Arg Phe Glu Glu Pro Val
   660 665 670
   Val Leu Pro Asp Leu Ser Asp Gln Thr Asp His Arg Gln Trp Thr Gln
   675 680 685
   Gln His Leu Asp Ala Ala Asp Leu Arg Met Ser Ala Met Ala Pro Thr
   690 695 700
   Pro Pro Gln Gly Glu Val Asp Ala Asp Asp Tyr Lys Asp Asp Asp Asp
   705 710 715 720
   Lys His His His His His His His His
   725
<210> 3
   <211> 525
   <212> DNA
   <213> Artificial sequence
<220>
   <223> Wildtype notch DNA sequence
<400> 3
   aatattcctt acaagattga ggccgtgaag agtgagccgg tggagcctcc gctgccctcg 60
   cagctgcacc tcatgtacgt ggcagcggcc gccttcgtgc tcctgttctt tgtgggctgt 120
   ggggtgctgc tgtcccgcaa gcgccggcgg cagcatggcc agctctggtt ccctgagggt 180
   ttcaaagtgt cagaggccag caagaagaag cggagagagc ccctcggcga ggactcagtc 240
   ggcctcaagc ccctgaagaa tgcctcagat ggtgctctga tggacgacaa tcagaacgag 300
   tggggagacg aagacctgga gaccaagaag ttccggtttg aggagccagt agttctccct 360
   gacctgagtg atcagactga ccacagacag tggacccagc agcacctgga cgctgctgac 420
   ctgcgcatgt ctgccatggc cccaacaccg cctcaggggg aggtggatgc tgacgattat 480
   aaagacgatg acgataaaca ccatcaccat caccatcacc attga 525
<210> 4
   <211> 174
   <212> PRT
   <213> Artificial sequence
<220>
   <223> Wildtype notch protein sequence
<400> 4
   Asn Ile Pro Tyr Lys Ile Glu Ala Val Lys Ser Glu Pro Val Glu Pro
   1               5                   10                  15
   Pro Leu Pro Ser Gln Leu His Leu Met Tyr Val Ala Ala Ala Ala Phe
               20                  25                  30
   Val Leu Leu Phe Phe Val Gly Cys Gly Val Leu Leu Ser Arg Lys Arg
           35                  40                  45
   Arg Arg Gln His Gly Gln Leu Trp Phe Pro Glu Gly Phe Lys Val Ser
       50                  55                  60
   Glu Ala Ser Lys Lys Lys Arg Arg Glu Pro Leu Gly Glu Asp Ser Val
   65                  70                  75                  80
   Gly Leu Lys Pro Leu Lys Asn Ala Ser Asp Gly Ala Leu Met Asp Asp
                   85                  90                  95
   Asn Gln Asn Glu Trp Gly Asp Glu Asp Leu Glu Thr Lys Lys Phe Arg
               100                 105                 110
   Phe Glu Glu Pro Val Val Leu Pro Asp Leu Ser Asp Gln Thr Asp His
           115                 120                 125
   Arg Gln Trp Thr Gln Gln His Leu Asp Ala Ala Asp Leu Arg Met Ser
       130                 135                 140
   Ala Met Ala Pro Thr Pro Pro Gln Gly Glu Val Asp Ala Asp Asp Tyr
   145                 150                 155                 160
   Lys Asp Asp Asp Asp Lys His His His His His His His His
                   165                 170
<210> 5
   <211> 2531
   <212> PRT
   <213> Mus musculus
<400> 5
   Met Pro Arg Leu Leu Thr Pro Leu Leu Cys Leu Thr Leu Leu Pro Ala
   1               5                   10                  15
   Arg Ala Ala Arg Gly Leu Arg Cys Ser Gln Pro Ser Gly Thr Cys Leu
               20                  25                  30
   Asn Gly Gly Arg Cys Glu Val Ala Ser Gly Thr Glu Ala Cys Val Ala
           35                  40                  45
   Ser Gly Ser Phe Val Gly Gln Arg Cys Gln Asp Pro Asn Pro Cys Leu
       50                  55                  60
   Ser Thr Arg Cys Lys Asn Ala Gly Thr Cys Tyr Val Val Asp His Gly
   65                  70                  75                  80
   Gly Ile Val Asp Tyr Ala Cys Ser Cys Pro Leu Gly Phe Ser Gly Pro
                   85                  90                  95
   Leu Cys Leu Thr Pro Leu Asp Lys Pro Cys Leu Ala Asn Pro Cys Arg
               100                 105                 110
   Asn Gly Gly Thr Cys Asp Leu Leu Thr Leu Thr Glu Tyr Lys Cys Arg
           115                 120                 125
   Cys Ser Pro Gly Trp Ser Gly Lys Ser Cys Gln Gln Ala Asp Pro Cys
   130 135 140
   Ala Ser Asn Pro Cys Ala Asn Gly Gly Gln Cys Leu Pro Phe Glu Ser
   145 150 155 160
   Ser Tyr Ile Cys Arg Cys Pro Pro Gly Phe His Gly Pro Thr Cys Arg
   165 170 175
   Gln Asp Val Asn Glu Cys Ser Gln Asn Pro Gly Leu Cys Arg His Gly
   180 185 190
   Gly His Cys His Asn Glu Ile Gly Ser Tyr Arg Cys Ala Cys Cys Ala
   195 200 205
   Thr His Thr Gly Pro His Cys Glu Leu Pro Tyr Val Pro Cys Ser Pro
   210 215 220
   Ser Pro Cys Gln Asn Gly Ala Thr Cys Arg Pro Thr Gly Asp Thr Thr
   225 230 235 240
   His Glu Cys Ala Cys Leu Pro Gly Phe Ala Gly Gln Asn Cys Glu Glu
   245 250 255
   Asn Val Asp Asp Cys Pro Gly Asn Asn Cys Lys Asn Gly Gly Ala Cys
   260 265 270
   Val Asp Gly Val Asn Thr Tyr Asn Cys Arg Cys Pro Pro Glu Val Thr
   275 280 285
   Gly Gln Tyr Cys Thr Glu Asp Val Asp Glu Cys Gln Leu Met Pro Asn
   290 295 300
   Ala Cys Gln Asn Ala Gly Thr Cys His Asn Thr His Gly Gly Tyr Asn
   305 310 315 320
   Cys Val Cys Val Asn Gly Trp Thr Gly Glu Asp Cys Ser Glu Asn Ile
   325 330 335
   Asp Asp Cys Ala Ser Ala Ala Cys Phe Gln Gly Ala Thr Cys His Asp
   340 345 350
   Arg Val Ala Ser Phe Tyr Cys Glu Cys Pro His Gly Arg Thr Gly Leu
   355 360 365
   Leu Cys His Leu Lys His Ala Cys Ile Ser Asn Pro Cys Asn Glu Gly
   370 375 380
   Ser Asn Cys Asp Thr Asn Pro Val Asn Gly Lys Arg Ile Cys Thr Cys
   385 390 395 400
   Pro Ser Gly Tyr Thr Gly Pro Ala Cys Ser Gln Asp Val Asp Glu Cys
   405 410 415
   Asp Leu Gly Ala Asn Arg Cys Glu His Ala Gly Lys Cys Leu Asn Thr
   420 425 430
   Leu Gly Ser Phe Glu Cys Gln Cys Leu Gln Gly Tyr Thr Gly Pro Gly
   435 440 445
   Cys Glu Ile Asp Val Asn Glu Cys Ile Ser Asn Pro Cys Gln Asn Asp
       450                 455                 460
   Ala Thr Cys Leu Asp Gln Ile Gly Glu Phe Gln Cys Ile Cys Met Pro
   465                 470                 475                 480
   Gly Tyr Glu Gly Val Tyr Cys Glu Ile Asn Thr Asp Glu Cys Ala Ser
                   485                 490                 495
   Ser Pro Cys Leu His Asn Gly His Cys Met Asp Lys Ile His Glu Phe
               500                 505                 510
   Gln Cys Gln Cys Pro Lys Gly Phe Asn Gly His Leu Cys Gln Tyr Asp
           515                 520                 525
   Val Asp Glu Cys Ala Ser Thr Pro Cys Lys Asn Gly Ala Lys Cys Leu
       530                 535                 540
   Asp Gly Pro Asn Thr Tyr Thr Cys Val Cys Thr Glu Gly Tyr Thr Gly
   545                 550                 555                 560
   Thr His Cys Glu Val Asp Ile Asp Glu Cys Asp Pro Asp Pro Cys His
                   565                 570                 575
   Tyr Gly Ser Cys Lys Asp Gly Val Ala Thr Phe Thr Cys Leu Cys Gln
               580                 585                 590
   Pro Gly Tyr Thr Gly His His Cys Glu Thr Asn Ile Asn Glu Cys His
           595                 600                 605
   Ser Gln Pro Cys Arg His Gly Gly Thr Cys Gln Asp Arg Asp Asn Ser
       610                 615                 620
   Tyr Leu Cys Leu Cys Leu Lys Gly Thr Thr Gly Pro Asn Cys Glu Ile
   625                 630                 635                 640
   Asn Leu Asp Asp Cys Ala Ser Asn Pro Cys Asp Ser Gly Thr Cys Leu
                   645                 650                 655
   Asp Lys Ile Asp Gly Tyr Glu Cys Ala Cys Glu Pro Gly Tyr Thr Gly
               660                 665                 670
   Ser Met Cys Asn Val Asn Ile Asp Glu Cys Ala Gly Ser Pro Cys His
           675                 680                 685
   Asn Gly Gly Thr Cys Glu Asp Gly Ile Ala Gly Phe Thr Cys Arg Cys
       690                 695                 700
   Pro Glu Gly Tyr His Asp Pro Thr Cys Leu Ser Glu Val Asn Glu Cys
   705                 710                 715                 720
   Asn Ser Asn Pro Cys Ile His Gly Ala Cys Arg Asp Gly Leu Asn Gly
                   725                 730                 735
   Tyr Lys Cys Asp Cys Ala Pro Gly Trp Ser Gly Thr Asn Cys Asp Ile
               740                 745                 750
   Asn Asn Asn Glu Cys Glu Ser Asn Pro Cys Val Asn Gly Gly Thr Cys
           755                 760                 765
   Lys Asp Met Thr Ser Gly Tyr Val Cys Thr Cys Arg Glu Gly Phe Ser
       770                 775                 780
   Gly Pro Asn Cys Gln Thr Asn Ile Asn Glu Cys Ala Ser Asn Pro Cys
   785 790 795 800
   Leu Asn Gln Gly Thr Cys Ile Asp Asp Val Ala Gly Tyr Lys Cys Asn
   805 810 815
   Cys Pro Leu Pro Tyr Thr Gly Ala Thr Cys Glu Val Val Leu Ala Pro
   820 825 830
   Cys Ala Thr Ser Pro Cys Lys Asn Ser Gly Val Cys Lys Glu Ser Glu
   835 840 845
   Asp Tyr Glu Ser Phe Ser Cys Val Cys Pro Thr Gly Trp Gln Gly Gln
   850 855 860
   Thr Cys Glu Val Asp Ile Asn Glu Cys Val Lys Ser Pro Cys Arg His
   865 870 875 880
   Gly Ala Ser Cys Gln Asn Thr Asn Gly Ser Tyr Arg Cys Leu Cys Gln
   885 890 895
   Ala Gly Tyr Thr Gly Arg Asn Cys Glu Ser Asp Ile Asp Asp Cys Arg
   900 905 910
   Pro Asn Pro Cys His Asn Gly Gly Ser Cys Thr Asp Gly Ile Asn Thr
   915 920 925
   Ala Phe Cys Asp Cys Leu Pro Gly Phe Gln Gly Ala Phe Cys Glu Glu
   930 935 940
   Asp Ile Asn Glu Cys Ala Ser Asn Pro Cys Gln Asn Gly Ala Asn Cys
   945 950 955 960
   Thr Asp Cys Val Asp Ser Tyr Thr Cys Thr Cys Pro Val Gly Phe Asn
   965 970 975
   Gly Ile His Cys Glu Asn Asn Thr Pro Asp Cys Thr Glu Ser Ser Cys
   980 985 990
   Phe Asn Gly Gly Thr Cys Val Asp Gly Ile Asn Ser Phe Thr Cys Leu
   995 1000 1005
   Cys Pro Pro Gly Phe Thr Gly Ser Tyr Cys Gln Tyr Asp Val Asn
   1010 1015 1020
   Glu Cys Asp Ser Arg Pro Cys Leu His Gly Gly Thr Cys Gln Asp
   1025 1030 1035
   Ser Tyr Gly Thr Tyr Lys Cys Thr Cys Pro Gln Gly Tyr Thr Gly
   1040 1045 1050
   Leu Asn Cys Gln Asn Leu Val Arg Trp Cys Asp Ser Ala Pro Cys
   1055 1060 1065
   Lys Asn Gly Gly Arg Cys Trp Gln Thr Asn Thr Gln Tyr His Cys
   1070 1075 1080
   Glu Cys Arg Ser Gly Trp Thr Gly Val Asn Cys Asp Val Leu Ser
   1085 1090 1095
   Val Ser Cys Glu Val Ala Ala Gln Lys Arg Gly Ile Asp Val Thr
   1100 1105 1110
   Leu Leu Cys Gln His Gly Gly Leu Cys Val Asp Glu Gly Asp Lys
   1115 1120 1125
   His Tyr Cys His Cys Gln Ala Gly Tyr Thr Gly Ser Tyr Cys Glu
   1130 1135 1140
   Asp Glu Val Asp Glu Cys Ser Pro Asn Pro Cys Gln Asn Gly Ala
   1145 1150 1155
   Thr Cys Thr Asp Tyr Leu Gly Gly Phe Ser Cys Lys Cys Val Ala
   1160 1165 1170
   Gly Tyr His Gly Ser Asn Cys Ser Glu Glu Ile Asn Glu Cys Leu
   1175 1180 1185
   Ser Gln Pro Cys Gln Asn Gly Gly Thr Cys Ile Asp Leu Thr Asn
   1190 1195 1200
   Ser Tyr Lys Cys Ser Cys Pro Arg Gly Thr Gln Gly Val His Cys
   1205 1210 1215
   Glu Ile Asn Val Asp Asp Cys His Pro Pro Leu Asp Pro Ala Ser
   1220 1225 1230
   Arg Ser Pro Lys Cys Phe Asn Asn Gly Thr Cys Val Asp Gln Val
   1235 1240 1245
   Gly Gly Tyr Thr Cys Thr Cys Pro Pro Gly Phe Val Gly Glu Arg
   1250 1255 1260
   Cys Glu Gly Asp Val Asn Glu Cys Leu Ser Asn Pro Cys Asp Pro
   1265 1270 1275
   Arg Gly Thr Gln Asn Cys Val Gln Arg Val Asn Asp Phe His Cys
   1280 1285 1290
   Glu Cys Arg Ala Gly His Thr Gly Arg Arg Cys Glu Ser Val Ile
   1295 1300 1305
   Asn Gly Cys Arg Gly Lys Pro Cys Lys Asn Gly Gly Val Cys Ala
   1310 1315 1320
   Val Ala Ser Asn Thr Ala Arg Gly Phe Ile Cys Arg Cys Pro Ala
   1325 1330 1335
   Gly Phe Glu Gly Ala Thr Cys Glu Asn Asp Ala Arg Thr Cys Gly
   1340 1345 1350
   Ser Leu Arg Cys Leu Asn Gly Gly Thr Cys Ile Ser Gly Pro Arg
   1355 1360 1365
   Ser Pro Thr Cys Leu Cys Leu Gly Ser Phe Thr Gly Pro Glu Cys
   1370 1375 1380
   Gln Phe Pro Ala Ser Ser Pro Cys Val Gly Ser Asn Pro Cys Tyr
   1385 1390 1395
   Asn Gln Gly Thr Cys Glu Pro Thr Ser Glu Asn Pro Phe Tyr Arg
   1400 1405 1410
   Cys Leu Cys Pro Ala Lys Phe Asn Gly Leu Leu Cys His Ile Leu
   1415 1420 1425
   Asp Tyr Ser Phe Thr Gly Gly Ala Gly Pro Asp Ile Pro Pro Pro
   1430 1435 1440
   Gln Ile Glu Glu Ala Cys Glu Leu Pro Glu Cys Gln Val Asp Ala
   1445 1450 1455
   Gly Asn Lys Val Cys Asn Leu Gln Cys Asn Asn His Ala Cys Gly
   1460 1465 1470
   Trp Asp Gly Gly Asp Cys Ser Leu Asn Phe Asn Asp Pro Trp Lys
   1475 1480 1485
   Asn Cys Thr Gln Ser Leu Gln Cys Trp Lys Tyr Phe Ser Asp Gly
   1490 1495 1500
   His Cys Asp Ser Gln Cys Asn Ser Ala Gly Cys Leu Phe Asp Gly
   1505 1510 1515
   Phe Asp Cys Gln Leu Thr Glu Gly Gln Cys Asn Pro Leu Tyr Asp
   1520 1525 1530
   Gln Tyr Cys Lys Asp His Phe Ser Asp Gly His Cys Asp Gln Gly
   1535 1540 1545
   Cys Asn Ser Ala Glu Cys Glu Trp Asp Gly Leu Asp Cys Ala Glu
   1550 1555 1560
   His Val Pro Glu Arg Leu Ala Ala Gly Thr Leu Val Leu Val Val
   1565 1570 1575
   Leu Leu Pro Pro Asp Gln Leu Arg Asn Asn Ser Phe His Phe Leu
   1580 1585 1590
   Arg Glu Leu Ser His Val Leu His Thr Asn Val Val Phe Lys Arg
   1595 1600 1605
   Asp Ala Gln Gly Gln Gln Met Ile Phe Pro Tyr Tyr Gly His Glu
   1610 1615 1620
   Glu Glu Leu Arg Lys His Pro Ile Lys Arg Ser Thr Val Gly Trp
   1625 1630 1635
   Ala Thr Ser Ser Leu Leu Pro Gly Thr Ser Gly Gly Arg Gln Arg
   1640 1645 1650
   Arg Glu Leu Asp Pro Met Asp Ile Arg Gly Ser Ile Val Tyr Leu
   1655 1660 1665
   Glu Ile Asp Asn Arg Gln Cys Val Gln Ser Ser Ser Gln Cys Phe
   1670 1675 1680
   Gln Ser Ala Thr Asp Val Ala Ala Phe Leu Gly Ala Leu Ala Ser
   1685 1690 1695
   Leu Gly Ser Leu Asn Ile Pro Tyr Lys Ile Glu Ala Val Lys Ser
   1700 1705 1710
   Glu Pro Val Glu Pro Pro Leu Pro Ser Gln Leu His Leu Met Tyr
       1715                1720                1725
   Val Ala Ala Ala Ala Phe Val Leu Leu Phe Phe Val Gly Cys Gly
       1730                1735                1740
   Val Leu Leu Ser Arg Lys Arg Arg Arg Gln His Gly Gln Leu Trp
       1745                1750                1755
   Phe Pro Glu Gly Phe Lys Val Ser Glu Ala Ser Lys Lys Lys Arg
       1760                1765                1770
   Arg Glu Pro Leu Gly Glu Asp Ser Val Gly Leu Lys Pro Leu Lys
       1775                1780                1785
   Asn Ala Ser Asp Gly Ala Leu Met Asp Asp Asn Gln Asn Glu Trp
       1790                1795                1800
   Gly Asp Glu Asp Leu Glu Thr Lys Lys Phe Arg Phe Glu Glu Pro
       1805                1810                1815
   Val Val Leu Pro Asp Leu Ser Asp Gln Thr Asp His Arg Gln Trp
       1820                1825                1830
   Thr Gln Gln His Leu Asp Ala Ala Asp Leu Arg Met Ser Ala Met
       1835                1840                1845
   Ala Pro Thr Pro Pro Gln Gly Glu Val Asp Ala Asp Cys Met Asp
       1850                1855                1860
   Val Asn Val Arg Gly Pro Asp Gly Phe Thr Pro Leu Met Ile Ala
       1865                1870                1875
   Ser Cys Ser Gly Gly Gly Leu Glu Thr Gly Asn Ser Glu Glu Glu
       1880                1885                1890
   Glu Asp Ala Pro Ala Val Ile Ser Asp Phe Ile Tyr Gln Gly Ala
       1895                1900                1905
   Ser Leu His Asn Gln Thr Asp Arg Thr Gly Glu Thr Ala Leu His
       1910                1915                1920
   Leu Ala Ala Arg Tyr Ser Arg Ser Asp Arg Arg Lys Arg Leu Glu
       1925                1930                1935
   Ala Ser Ala Asp Ala Asn Ile Gln Asp Asn Met Gly Arg Thr Pro
       1940                1945                1950
   Leu His Ala Ala Val Ser Ala Asp Ala Gln Gly Val Phe Gln Ile
       1955                1960                1965
   Leu Leu Arg Asn Arg Ala Thr Asp Leu Asp Ala Arg Met His Asp
       1970                1975                1980
   Gly Thr Thr Pro Leu Ile Leu Ala Ala Arg Leu Ala Val Glu Gly
       1985                1990                1995
   Met Leu Glu Asp Leu Ile Asn Ser His Ala Asp Val Asn Ala Val
       2000                2005                2010
   Asp Asp Leu Gly Lys Ser Ala Leu His Trp Ala Ala Ala Val Asn
       2015                2020                2025
   Asn Val Asp Ala Ala Val Val Leu Leu Lys Asn Gly Ala Asn Lys
   2030 2035 2040
   Asp Ile Glu Asn Asn Lys Glu Glu Thr Ser Leu Phe Leu Ser Ile
   2045 2050 2055
   Arg Arg Glu Ser Tyr Glu Thr Ala Lys Val Leu Leu Asp His Phe
   2060 2065 2070
   Ala Asn Arg Asp Ile Thr Asp His Met Asp Arg Leu Pro Arg Asp
   2075 2080 2085
   Ile Ala Gln Glu Arg Met His His Asp Ile Val Arg Leu Leu Asp
   2090 2095 2100
   Glu Tyr Asn Leu Val Arg Ser Pro Gln Leu His Gly Thr Ala Leu
   2105 2110 2115
   Gly Gly Thr Pro Thr Leu Ser Pro Thr Leu Cys Ser Pro Asn Gly
   2120 2125 2130
   Tyr Pro Gly Asn Leu Lys Ser Ala Thr Gln Gly Lys Lys Ala Arg
   2135 2140 2145
   Lys Pro Ser Thr Lys Gly Leu Ala Cys Gly Ser Lys Glu Ala Lys
   2150 2155 2160
   Asp Leu Lys Ala Arg Arg Lys Ser Ser Gln Asp Gly Lys Gly Trp
   2165 2170 2175
   Leu Leu Asp Ser Ser Ser Ser Met Leu Ser Pro Val Asp Ser Leu
   2180 2185 2190
   Glu Ser Pro His Gly Tyr Leu Ser Asp Val Ala Ser His Pro Leu
   2195 2200 2205
   Leu Pro Ser Pro Phe Gln Gln Ser Pro Ser Met Pro Leu Ser His
   2210 2215 2220
   Leu Pro Gly Met Pro Asp Thr His Leu Gly Ile Ser His Leu Asn
   2225 2230 2235
   Val Ala Ala Lys Pro Glu Met Ala Ala Leu Ala Gly Gly Ser Arg
   2240 2245 2250
   Leu Ala Phe Glu His Pro Pro Pro Arg Leu Ser His Leu Pro Val
   2255 2260 2265
   Ala Ser Ser Ala Cys Thr Val Leu Ser Thr Asn Gly Thr Gly Ala
   2270 2275 2280
   Met Asn Phe Thr Val Gly Ala Pro Ala Ser Leu Asn Gly Gln Cys
   2285 2290 2295
   Glu Trp Leu Pro Arg Leu Gln Asn Gly Met Val Pro Ser Gln Tyr
   2300 2305 2310
   Asn Pro Leu Arg Pro Gly Val Thr Pro Gly Thr Leu Ser Thr Gln
   2315 2320 2325
   Ala Ala Gly Leu Gln His Ser Met Met Gly Pro Leu His Ser Ser
       2330                2335                2340
   Leu Ser Thr Asn Thr Leu Ser Pro Ile Ile Tyr Gln Gly Leu Pro
       2345                2350                2355
   Asn Thr Arg Leu Ala Thr Gln Pro His Leu Val Gln Thr Gln Gln
       2360                2365                2370
   Val Gln Pro Gln Asn Leu Pro Leu Gln Pro Gln Asn Leu Gln Pro
       2375                2380                2385
   Pro Ser Gln Pro His Leu Ser Val Ser Ser Ala Ala Asn Gly His
       2390                2395                2400
   Leu Gly Arg Ser Phe Leu Ser Gly Glu Pro Ser Gln Ala Asp Val
       2405                2410                2415
   Gln Pro Leu Gly Pro Ser Ser Leu Pro Val His Thr Ile Leu Pro
       2420                2425                2430
   Gln Glu Ser Gln Ala Leu Pro Thr Ser Leu Pro Ser Ser Met Val
       2435                2440                2445
   Pro Pro Met Thr Thr Thr Gln Phe Leu Thr Pro Pro Ser Gln His
       2450                2455                2460
   Ser Tyr Ser Ser Ser Pro Val Asp Asn Thr Pro Ser His Gln Leu
       2465                2470                2475
   Gln Val Pro Glu Pro Thr Phe Leu Thr Pro Ser Pro Glu Ser Pro
       2480                2485                2490
   Asp Gln Trp Ser Ser Ser Ser Pro His Ser Asn Ile Ser Asp Trp
       2495                2500                2505
   Ser Glu Gly Ile Ser Ser Pro Pro Thr Thr Met Pro Ser Gln Ile
       2510                2515                2520
   Thr His Ile Pro Glu Ala Phe Lys
       2525                2530
<210> 6
   <211> 2444
   <212> PRT
   <213> Homo sapiens
<220>
   <221> misc_feature
   <222> (891)..(891)
   <223> Xaa = Any or unknown amino acid
<220>
   <221> misc_feature
   <222> (1763)..(1763)
   <223> Xaa = Any or unknown amino acid
<220>
   <221> misc_feature
   <222> (1787)..(1787)
   <223> Xaa = Any or unknown amino acid
<400> 6
   Met Pro Pro Leu Leu Ala Pro Leu Leu Cys Leu Ala Leu Leu Pro Ala
   1               5                   10                  15
   Leu Ala Ala Arg Gly Pro Arg Cys Ser Gln Pro Gly Glu Thr Cys Leu
               20                  25                  30
   Asn Gly Gly Lys Cys Glu Ala Ala Asn Gly Thr Glu Ala Cys Val Cys
           35                  40                  45
   Gly Gly Ala Phe Val Gly Pro Arg Cys Gln Asp Pro Asn Pro Cys Leu
       50                  55                  60
   Ser Thr Pro Cys Lys Asn Ala Gly Thr Cys His Val Val Asp Arg Arg
   65                  70                  75                  80
   Gly Val Ala Asp Tyr Ala Cys Ser Cys Ala Leu Gly Phe Ser Gly Pro
                   85                  90                  95
   Leu Cys Leu Thr Pro Leu Asp Asn Ala Cys Leu Thr Asn Pro Cys Arg
               100                 105                 110
   Asn Gly Gly Thr Cys Asp Leu Leu Thr Leu Thr Glu Tyr Lys Cys Arg
           115                 120                 125
   Cys Pro Pro Gly Trp Ser Gly Lys Ser Cys Gln Gln Ala Asp Pro Cys
       130                 135                 140
   Ala Ser Asn Pro Cys Ala Asn Gly Gly Gln Cys Leu Pro Phe Glu Ala
   145                 150                 155                 160
   Ser Tyr Ile Cys His Cys Pro Pro Ser Phe His Gly Pro Thr Cys Arg
                   165                 170                 175
   Gln Asp Val Asn Glu Cys Gly Gln Lys Pro Arg Leu Cys Arg His Gly
               180                 185                 190
   Gly Thr Cys His Asn Glu Val Gly Ser Tyr Arg Cys Val Cys Arg Ala
           195                 200                 205
   Thr His Thr Gly Pro Asn Cys Glu Arg Pro Tyr Val Pro Cys Ser Pro
       210                 215                 220
   Ser Pro Cys Gln Asn Gly Gly Thr Cys Arg Pro Thr Gly Asp Val Thr
   225                 230                 235                 240
   His Glu Cys Ala Cys Leu Pro Gly Phe Thr Gly Gln Asn Cys Glu Glu
                   245                 250                 255
   Asn Ile Asp Asp Cys Pro Gly Asn Asn Cys Lys Asn Gly Gly Ala Cys
               260                 265                 270
   Val Asp Gly Val Asn Thr Tyr Asn Cys Pro Cys Pro Pro Glu Trp Thr
           275                 280                 285
   Gly Gln Tyr Cys Thr Glu Asp Val Asp Glu Cys Gln Leu Met Pro Asn
       290                 295                 300
   Ala Cys Gln Asn Gly Gly Thr Cys His Asn Thr His Gly Gly Tyr Asn
   305                 310                 315                 320
   Cys Val Cys Val Asn Gly Trp Thr Gly Glu Asp Cys Ser Glu Asn Ile
                   325                 330                 335
   Asp Asp Cys Ala Ser Ala Ala Cys Phe His Gly Ala Thr Cys His Asp
               340                 345                 350
   Arg Val Ala Ser Phe Tyr Cys Glu Cys Pro His Gly Arg Thr Gly Leu
           355                 360                 365
   Leu Cys His Leu Asn Asp Ala Cys Ile Ser Asn Pro Cys Asn Glu Gly
       370                 375                 380
   Ser Asn Cys Asp Thr Asn Pro Val Asn Gly Lys Ala Ile Cys Thr Cys
   385                 390                 395                 400
   Pro Ser Gly Tyr Thr Gly Pro Ala Cys Ser Gln Asp Val Asp Glu Cys
                   405                 410                 415
   Ser Leu Gly Ala Asn Pro Cys Glu His Ala Gly Lys Cys Ile Asn Thr
               420                 425                 430
   Leu Gly Ser Phe Glu Cys Gln Cys Leu Gln Gly Tyr Thr Gly Pro Arg
           435                 440                 445
   Cys Glu Ile Asp Val Asn Glu Cys Val Ser Asn Pro Cys Gln Asn Asp
       450                 455                 460
   Ala Thr Cys Leu Asp Gln Ile Gly Glu Phe Gln Cys Met Cys Met Pro
   465                 470                 475                 480
   Gly Tyr Glu Gly Val His Cys Glu Val Asn Thr Asp Glu Cys Ala Ser
                   485                 490                 495
   Ser Pro Cys Leu His Asn Gly Arg Cys Leu Asp Lys Ile Asn Glu Phe
               500                 505                 510
   Gln Cys Glu Cys Pro Thr Gly Phe Thr Gly His Leu Cys Gln Tyr Asp
           515                 520                 525
   Val Asp Glu Cys Ala Ser Thr Pro Cys Lys Asn Gly Ala Lys Cys Leu
       530                 535                 540
   Asp Gly Pro Asn Thr Tyr Thr Cys Val Cys Thr Glu Gly Tyr Thr Gly
   545                 550                 555                 560
   Thr His Cys Glu Val Asp Ile Asp Glu Cys Asp Pro Asp Pro Cys His
                   565                 570                 575
   Tyr Gly Ser Cys Lys Asp Gly Val Ala Thr Phe Thr Cys Leu Cys Arg
               580                 585                 590
   Pro Gly Tyr Thr Gly His His Cys Glu Thr Asn Ile Asn Glu Cys Ser
           595                 600                 605
   Ser Gln Pro Cys Arg Leu Arg Gly Thr Cys Gln Asp Pro Asp Asn Ala
       610                 615                 620
   Tyr Leu Cys Phe Cys Leu Lys Gly Thr Thr Gly Pro Asn Cys Glu Ile
   625                 630                 635                 640
   Asn Leu Asp Asp Cys Ala Ser Ser Pro Cys Asp Ser Gly Thr Cys Leu
                   645                 650                 655
   Asp Lys Ile Asp Gly Tyr Glu Cys Ala Cys Glu Pro Gly Tyr Thr Gly
               660                 665                 670
   Ser Met Cys Asn Ser Asn Ile Asp Glu Cys Ala Gly Asn Pro Cys His
           675                 680                 685
   Asn Gly Gly Thr Cys Glu Asp Gly Ile Asn Gly Phe Thr Cys Arg Cys
       690                 695                 700
   Pro Glu Gly Tyr His Asp Pro Thr Cys Leu Ser Glu Val Asn Glu Cys
   705                 710                 715                 720
   Asn Ser Asn Pro Cys Val His Gly Ala Cys Arg Asp Ser Leu Asn Gly
                   725                 730                 735
   Tyr Lys Cys Asp Cys Asp Pro Gly Trp Ser Gly Thr Asn Cys Asp Ile
               740                 745                 750
   Asn Asn Asn Glu Cys Glu Ser Asn Pro Cys Val Asn Gly Gly Thr Cys
           755                 760                 765
   Lys Asp Met Thr Ser Gly Ile Val Cys Thr Cys Arg Glu Gly Phe Ser
       770                 775                 780
   Gly Pro Asn Cys Gln Thr Asn Ile Asn Glu Cys Ala Ser Asn Pro Cys
   785                 790                 795                 800
   Leu Asn Lys Gly Thr Cys Ile Asp Asp Val Ala Gly Tyr Lys Cys Asn
                   805                 810                 815
   Cys Leu Leu Pro Tyr Thr Gly Ala Thr Cys Glu Val Val Leu Ala Pro
               820                 825                 830
   Cys Ala Pro Ser Pro Cys Arg Asn Gly Gly Glu Cys Arg Gln Ser Glu
           835                 840                 845
   Asp Tyr Glu Ser Phe Ser Cys Val Cys Pro Thr Ala Gly Ala Lys Gly
       850                 855                 860
   Gln Thr Cys Glu Val Asp Ile Asn Glu Cys Val Leu Ser Pro Cys Arg
   865                 870                 875                 880
   His Gly Ala Ser Cys Gln Asn Thr His Gly Xaa Tyr Arg Cys His Cys
                   885                 890                 895
   Gln Ala Gly Tyr Ser Gly Arg Asn Cys Glu Thr Asp Ile Asp Asp Cys
               900                 905                 910
   Arg Pro Asn Pro Cys His Asn Gly Gly Ser Cys Thr Asp Gly Ile Asn
           915                 920                 925
   Thr Ala Phe Cys Asp Cys Leu Pro Gly Phe Arg Gly Thr Phe Cys Glu
       930                 935                 940
   Glu Asp Ile Asn Glu Cys Ala Ser Asp Pro Cys Arg Asn Gly Ala Asn
   945                 950                 955                 960
   Cys Thr Asp Cys Val Asp Ser Tyr Thr Cys Thr Cys Pro Ala Gly Phe
                   965                 970                 975
   Ser Gly Ile His Cys Glu Asn Asn Thr Pro Asp Cys Thr Glu Ser Ser
               980                 985                 990
   Cys Phe Asn Gly Gly Thr Cys Val Asp Gly Ile Asn Ser Phe Thr Cys
   995 1000 1005
   Leu Cys Pro Pro Gly Phe Thr Gly Ser Tyr Cys Gln His Val Val
   1010 1015 1020
   Asn Glu Cys Asp Ser Arg Pro Cys Leu Leu Gly Gly Thr Cys Gln
   1025 1030 1035
   Asp Gly Arg Gly Leu His Arg Cys Thr Cys Pro Gln Gly Tyr Thr
   1040 1045 1050
   Gly Pro Asn Cys Gln Asn Leu Val His Trp Cys Asp Ser Ser Pro
   1055 1060 1065
   Cys Lys Asn Gly Gly Lys Cys Trp Gln Thr His Thr Gln Tyr Arg
   1070 1075 1080
   Cys Glu Cys Pro Ser Gly Trp Thr Gly Leu Tyr Cys Asp Val Pro
   1085 1090 1095
   Ser Val Ser Cys Glu Val Ala Ala Gln Arg Gln Gly Val Asp Val
   1100 1105 1110
   Ala Arg Leu Cys Gln His Gly Gly Leu Cys Val Asp Ala Gly Asn
   1115 1120 1125
   Thr His His Cys Arg Cys Gln Ala Gly Tyr Thr Gly Ser Tyr Cys
   1130 1135 1140
   Glu Asp Leu Val Asp Glu Cys Ser Pro Ser Pro Cys Gln Asn Gly
   1145 1150 1155
   Ala Thr Cys Thr Asp Tyr Leu Gly Gly Tyr Ser Cys Lys Cys Val
   1160 1165 1170
   Ala Gly Tyr His Gly Val Asn Cys Ser Glu Glu Ile Asp Glu Cys
   1175 1180 1185
   Leu Ser His Pro Cys Gln Asn Gly Gly Thr Cys Leu Asp Leu Pro
   1190 1195 1200
   Asn Thr Tyr Lys Cys Ser Cys Pro Arg Gly Thr Gln Gly Val His
   1205 1210 1215
   Cys Glu Ile Asn Val Asp Asp Cys Asn Pro Pro Val Asp Pro Val
   1220 1225 1230
   Ser Arg Ser Pro Lys Cys Phe Asn Asn Gly Thr Cys Val Asp Gln
   1235 1240 1245
   Val Gly Gly Tyr Ser Cys Thr Cys Pro Pro Gly Phe Val Gly Glu
   1250 1255 1260
   Arg Cys Glu Gly Asp Val Asn Glu Cys Leu Ser Asn Pro Cys Asp
   1265 1270 1275
   Ala Arg Gly Thr Gln Asn Cys Val Gln Arg Val Asn Asp Phe His
   1280 1285 1290
   Cys Glu Cys Arg Ala Gly His Thr Gly Arg Arg Cys Glu Ser Val
           1295                            1300                            1305
   Ile Asn Gly Cys Lys Gly Lys Pro Cys Lys Asn Gly Gly Thr Cys
       1310                    1315                    1320
   Ala Val Ala Ser Asn Thr Ala Arg Gly Phe Ile Cys Lys Cys Pro
       1325                    1330                    1335
   Ala Gly Phe Glu Gly Ala Thr Cys Glu Asn Asp Ala Arg Thr Cys
       1340                    1345                    1350
   Gly Ser Leu Arg Cys Leu Asn Gly Gly Thr Cys Ile Ser Gly Pro
       1355                    1360                    1365
   Arg Ser Pro Thr Cys Leu Cys Leu Gly Pro Phe Thr Gly Pro Glu
       1370                    1375                    1380
   Cys Gln Phe Pro Ala Ser Ser Pro Cys Leu Gly Gly Asn Pro Cys
       1385                    1390                    1395
   Tyr Asn Gln Gly Thr Cys Glu Pro Thr Ser Glu Ser Pro Phe Tyr
       1400                    1405                    1410
   Arg Cys Leu Cys Pro Ala Lys Phe Asn Gly Leu Leu Cys His Ile
       1415                    1420                    1425
   Leu Asp Tyr Ser Phe Gly Gly Gly Ala Gly Arg Asp Ile Pro Pro
       1430                    1435                    1440
   Pro Leu Ile Glu Glu Ala Cys Glu Leu Pro Glu Cys Gln Glu Asp
       1445                    1450                    1455
   Ala Gly Asn Lys Val Cys Ser Leu Gln Cys Asn Asn His Ala Cys
       1460                    1465                    1470
   Gly Trp Asp Gly Gly Asp Cys Ser Leu Asn Phe Asn Asp Pro Trp
       1475                    1480                    1485
   Lys Asn Cys Thr Gln Ser Leu Gln Cys Trp Lys Tyr Phe Ser Asp
       1490                    1495                    1500
   Gly His Cys Asp Ser Gln Cys Asn Ser Ala Gly Cys Leu Phe Asp
       1505                    1510                    1515
   Gly Phe Asp Cys Gln Arg Ala Glu Gly Gln Cys Asn Pro Leu Tyr
       1520                    1525                    1530
   Asp Gln Tyr Cys Lys Asp His Phe Ser Asp Gly His Cys Asp Gln
       1535                    1540                    1545
   Gly Cys Asn Ser Ala Glu Cys Glu Trp Asp Gly Leu Asp Cys Ala
       1550                    1555                    1560
   Glu His Val Pro Glu Arg Leu Ala Ala Gly Thr Leu Val Val Val
       1565                    1570                    1575
   Val Leu Met Pro Pro Glu Gln Leu Arg Asn Ser Ser Phe His Phe
       1580                    1585                    1590
   Leu Arg Glu Leu Ser Arg Val Leu His Thr Asn Val Val Phe Lys
       1595                    1600                    1605
   Arg Asp Ala His Gly Gln Gln Met Ile Phe Pro Tyr Tyr Gly Arg
   1610 1615 1620
   Glu Glu Glu Leu Arg Lys His Pro Ile Lys Arg Ala Ala Glu Gly
   1625 1630 1635
   Trp Ala Ala Pro Asp Ala Leu Leu Gly Gln Val Lys Ala Ser Leu
   1640 1645 1650
   Leu Pro Gly Gly Ser Glu Gly Gly Arg Arg Arg Arg Glu Leu Asp
   1655 1660 1665
   Pro Met Asp Val Arg Gly Ser Ile Val Tyr Leu Glu Ile Asp Asn
   1670 1675 1680
   Arg Gln Cys Val Gln Ala Ser Ser Gln Cys Phe Gln Ser Ala Thr
   1685 1690 1695
   Asp Val Ala Ala Phe Leu Gly Ala Leu Ala Ser Leu Gly Ser Leu
   1700 1705 1710
   Asn Ile Pro Tyr Lys Ile Glu Ala Val Gln Ser Glu Thr Val Glu
   1715 1720 1725
   Pro Pro Pro Pro Ala Gln Leu His Phe Met Tyr Val Ala Ala Ala
   1730 1735 1740
   Ala Phe Val Leu Leu Phe Phe Val Gly Cys Gly Val Leu Leu Ser
   1745 1750 1755
   Arg Lys Arg Arg Xaa Gln His Gly Gln Leu Trp Phe Pro Glu Gly
   1760 1765 1770
   Phe Lys Val Ser Glu Ala Ser Lys Lys Lys Arg Arg Glu Xaa Leu
   1775 1780 1785
   Gly Glu Asp Ser Val Gly Leu Lys Pro Leu Lys Asn Ala Ser Asp
   1790 1795 1800
   Gly Ala Leu Met Asp Asp Asn Gln Asn Glu Trp Gly Asp Glu Asp
   1805 1810 1815
   Leu Glu Thr Lys Lys Phe Arg Phe Glu Glu Pro Val Val Leu Pro
   1820 1825 1830
   Asp Leu Asp Asp Gln Thr Asp His Arg Gln Trp Thr Gln Gln His
   1835 1840 1845
   Leu Asp Ala Ala Asp Leu Arg Met Ser Ala Met Ala Pro Thr Pro
   1850 1855 1860
   Pro Gln Gly Glu Val Asp Ala Asp Cys Met Asp Val Asn Val Arg
   1865 1870 1875
   Gly Pro Asp Gly Phe Thr Pro Leu Met Ile Ala Ser Cys Ser Gly
   1880 1885 1890
   Gly Gly Leu Glu Thr Gly Asn Ser Glu Glu Glu Glu Asp Ala Pro
   1895 1900 1905
   Ala Val Ile Ser Asp Phe Ile Tyr Gln Gly Ala Ser Leu His Asn
   1910 1915 1920
   Gln Thr Asp Arg Thr Gly Glu Thr Ala Leu His Leu Ala Ala Arg
   1925 1930 1935
   Tyr Ser Arg Ser Asp Ala Ala Lys Arg Leu Leu Glu Ala Ser Ala
   1940 1945 1950
   Asp Ala Asn Ile Gln Asp Asn Met Gly Arg Thr Pro Leu His Ala
   1955 1960 1965
   Ala Val Ser Ala Asp Ala Gln Gly Val Phe Gln Ile Leu Ile Arg
   1970 1975 1980
   Asn Arg Ala Thr Asp Leu Asp Ala Arg Met His Asp Gly Thr Thr
   1985 1990 1995
   Pro Leu Ile Leu Ala Ala Arg Leu Ala Val Glu Gly Met Leu Glu
   2000 2005 2010
   Asp Leu Ile Asn Ser His Ala Asp Val Asn Ala Val Asp Asp Leu
   2015 2020 2025
   Gly Lys Ser Ala Leu His Trp Ala Ala Ala Val Asn Asn Val Asp
   2030 2035 2040
   Ala Ala Val Val Leu Leu Lys Asn Gly Ala Asn Lys Asp Met Gln
   2045 2050 2055
   Asn Asn Arg Glu Glu Thr Pro Leu Phe Leu Ala Ala Arg Glu Gly
   2060 2065 2070
   Ser Tyr Glu Thr Ala Lys Val Leu Leu Asp His Phe Ala Asn Arg
   2075 2080 2085
   Asp Ile Thr Asp His Met Asp Arg Leu Pro Arg Asp Ile Ala Gln
   2090 2095 2100
   Glu Arg Met His His Asp Ile Val Arg Leu Leu Asp Glu Tyr Asn
   2105 2110 2115
   Leu Val Arg Ser Pro Gln Leu His Gly Ala Pro Leu Gly Gly Thr
   2120 2125 2130
   Pro Thr Leu Ser Pro Pro Leu Cys Ser Pro Asn Gly Tyr Leu Gly
   2135 2140 2145
   Ser Leu Lys Pro Gly Val Gln Gly Lys Lys Val Arg Lys Pro Ser
   2150 2155 2160
   Ser Lys Gly Leu Ala Cys Gly Ser Lys Glu Ala Lys Asp Leu Lys
   2165 2170 2175
   Ala Arg Arg Lys Lys Ser Gln Asp Gly Lys Gly Cys Leu Leu Asp
   2180 2185 2190
   Ser Ser Gly Met Leu Ser Pro Val Asp Ser Leu Glu Ser Pro His
   2195 2200 2205
   Gly Tyr Leu Ser Asp Val Ala Ser Pro Pro Leu Leu Pro Ser Pro
   2210 2215 2220
   Phe Gln  Gln Ser Pro Ser Val  Pro Leu Asn His Leu  Pro Gly Met
       2225                 2230                 2235
   Pro Asp  Thr His Leu Gly Ile  Gly His Leu Asn Val  Ala Ala Lys
       2240                 2245                 2250
   Pro Glu  Met Ala Ala Leu Gly  Gly Gly Gly Arg Leu  Ala Phe Glu
       2255                 2260                 2265
   Thr Gly  Pro Pro Arg Leu Ser  His Leu Pro Val Ala  Ser Gly Thr
       2270                 2275                 2280
   Ser Thr  Val Leu Gly Ser Ser  Ser Gly Gly Ala Leu  Asn Phe Thr
       2285                 2290                 2295
   Val Gly  Gly Ser Thr Ser Leu  Asn Gly Gln Cys Glu  Trp Leu Ser
       2300                 2305                 2310
   Arg Leu  Gln Ser Gly Met Val  Pro Asn Gln Tyr Asn  Pro Leu Arg
       2315                 2320                 2325
   Gly Ser  Val Ala Pro Gly Pro  Leu Ser Thr Gln Ala  Pro Ser Leu
       2330                 2335                 2340
   Gln His  Gly Met Val Gly Pro  Leu His Ser Ser Leu  Ala Ala Ser
       2345                 2350                 2355
   Ala Leu  Ser Gln Met Met Ser  Tyr Gln Gly Leu Pro  Ser Thr Arg
       2360                 2365                 2370
   Leu Ala  Thr Gln Pro His Leu  Val Gln Thr Gln Gln  Val Gln Pro
       2375                 2380                 2385
   Gln Asn  Leu Gln Met Gln Gln  Gln Asn Leu Gln Pro  Ala Asn Ile
       2390                 2395                 2400
   Gln Gln  Gln Gln Ser Leu Gln  Pro Pro Pro Pro Pro  Pro Gln Pro
       2405                 2410                 2415
   His Leu  Gly Val Ser Ser Ala  Ala Ser Gly His Leu  Gly Arg Ser
       2420                 2425                 2430
   Phe Leu  Ser Gly Glu Pro Ser  Gln Ala Asp Val
       2435                 2440
<210> 7
   <211>
   <212>
   <213>
<220>
   <223>
<400> 7
   deleted
<210> 8
<211>
   <212>
   <213>
   <220>
   <223>
<400> 8
   deleted
<210> 9
   <211>
   <212>
   <213>
<220>
   <223>
<400> 9
   deleted
<210> 10
   <211> 30
   <212> PRT
   <213> Artificial sequence
<220>
   <223> Amino acid sequence surrounding the transmembrane domains of APP
<400> 10
   Ser Asn Lys Gly Ala Ile Ile Gly Leu Met Val Gly Gly Val Val Ile
   1               5                   10                  15
   Ala Thr Val Ile Val Ile Thr Leu Val Met Leu Lys Lys Lys
               20                  25                  30
<210> 11
   <211> 30
   <212> PRT
   <213> Artificial sequence
<220>
   <223> Sequence surrounding the transmembrane domains of E-cathedrin
<400> 11
   Ile Pro Glu Trp Leu Ile Ile Leu Ala Ser Leu Leu Leu Ala Leu Ala
   1               5                   10                  15
   Leu Ile Leu Ala Val Cys Ile Ala Val Asn Ser Arg Arg Arg
               20                  25                  30
<210> 12
   <211> 30
   <212> PRT
   <213> Artificial sequence
<220>
   <223> Sequence surrounding the transmembrane domains of Notch-1
<400> 12
   Pro Ser Gln Leu His Leu Met Tyr Val Ala Ala Ala Ala Phe Val Leu
   1               5                   10                  15
   Leu Phe Phe Val Gly Cys Gly Val Leu Leu Ser Arg Lys Arg
               20                  25                  30
<210> 13
   <211> 158
   <212> PRT
   <213> Artificial sequence
<220>
   <223> Sequence surrounding the transmembrane domains of Notch-1
<400> 13
   Asn Ile Pro Tyr Lys Ile Glu Ala Val Lys Ser Glu Pro Val Glu Pro
   1               5                   10                  15
   Pro Leu Pro Ser Gln Leu His Leu Met Tyr Val Ala Ala Ala Ala Phe
               20                  25                  30
   Val Leu Leu Phe Phe Val Gly Cys Gly Val Leu Leu Ser Arg Lys Arg
           35                  40                  45
   Arg Arg Gln His Gly Gln Leu Trp Phe Pro Glu Gly Phe Lys Val Ser
       50                  55                  60
   Glu Ala Ser Lys Lys Lys Arg Arg Glu Pro Leu Gly Glu Asp Ser Val
   65                  70                  75                  80
   Gly Leu Lys Pro Leu Lys Asn Ala Ser Asp Gly Ala Leu Met Asp Asp
                   85                  90                  95
   Asn Gln Asn Glu Trp Gly Asp Glu Asp Leu Glu Thr Lys Lys Phe Arg
               100                 105                 110
   Phe Glu Glu Pro Val Val Leu Pro Asp Leu Ser Asp Gln Thr Asp His
           115                 120                 125
   Arg Gln Trp Thr Gln Gln His Leu Asp Ala Ala Asp Leu Arg Met Ser
       130                 135                 140
   Ala Met Ala Pro Thr Pro Pro Gln Gly Glu Val Asp Ala Asp
   145                 150                 155
<210> 14
   <211> 8
   <212> PRT
   <213> Artificial sequence
<220>
   <223> C-terminal flag sequence
<400> 14
   Asp Tyr Lys Asp Asp Asp Asp Lys
   1 5
<210> 15
   <211> 16
   <212> PRT
   <213> Artificial sequence
<220>
   <223> Flag/8 his tag
<400> 15
   Asp Tyr Lys Asp Asp Asp Asp Lys His His His His His His His His
   1 5 10 15
<210> 16
   <211> 1665
   <212> DNA
   <213> Artificial sequence
<220>
   <223> Nucleic acid encoding NusA
<400> 16
   atgaacaaag aaattttggc tgtagttgaa gccgtatcca atgaaaaggc gctacctcgc 60
   gagaagattt tcgaagcatt ggaaagcgcg ctggcgacag caacaaagaa aaaatatgaa 120
   caagagatcg acgtccgcgt acagatcgat cgcaaaagcg gtgattttga cactttccgt 180
   cgctggttag ttgttgatga agtcacccag ccgaccaagg aaatcaccct tgaagccgca 240
   cgttatgaag atgaaagcct gaacctgggc gattacgttg aagatcagat tgagtctgtt 300
   acctttgacc gtatcactac ccagacggca aaacaggtta tcgtgcagaa agtgcgtgaa 360
   gccgaacgtg cgatggtggt tgatcagttc cgtgaacacg aaggtgaaat catcaccggc    420
   gtggtgaaaa aagtaaaccg cgacaacatc tctctggatc tgggcaacaa cgctgaagcc    480
   gtgatcctgc gcgaagatat gctgccgcgt gaaaacttcc gccctggcga ccgcgttcgt    540
   ggcgtgctct attccgttcg cccggaagcg cgtggcgcgc aactgttcgt cactcgttcc    600
   aagccggaaa tgctgatcga actgttccgt attgaagtgc cagaaatcgg cgaagaagtg    660
   attgaaatta aagcagcggc tcgcgatccg ggttctcgtg cgaaaatcgc ggtgaaaacc    720
   aacgataaac gtatcgatcc ggtaggtgct tgcgtaggta tgcgtggcgc gcgtgttcag    780
   gcggtgtcta ctgaactggg tggcgagcgt atcgatatcg tcctgtggga tgataacccg    840
   gcgcagttcg tgattaacgc aatggcaccg gcagacgttg cttctatcgt ggtggatgaa    900
   gataaacaca ccatggacat cgccgttgaa gccggtaatc tggcgcaggc gattggccgt    960
   aacggtcaga acgtgcgtct ggcttcgcaa ctgagcggtt gggaactcaa cgtgatgacc   1020
   gttgacgacc tgcaagctaa gcatcaggcg gaagcgcacg cagcgatcga caccttcacc   1080
   aaatatctcg acatcgacga agacttcgcg actgttctgg tagaagaagg cttctcgacg   1140
   ctggaagaat tggcctatgt gccgatgaaa gagctgttgg aaatcgaagg ccttgatgag   1200
   ccgaccgttg aagcactgcg cgagcgtgct aaaaatgcac tggccaccat tgcacaggcc   1260
   caggaagaaa gcctcggtga taacaaaccg gctgacgatc tgctgaacct tgaaggggta   1320
   gatcgtgatt tggcattcaa actggccgcc cgtggcgttt gtacgctgga agatctcgcc   1380
   gaacagggca ttgatgatct ggctgatatc gaagggttga ccgacgaaaa agccggagca   1440
   ctgattatgg ctgcccgtaa tatttgctgg ttcggtgacg aagcgactag tggttctggt   1500
   catcaccatc accatcactc cgcgggtaaa gaaaccgctg ctgcgaaatt tgaacgccag   1560
   cacatggact cgccaccgcc aactggtctg gtcccccggg gcagcgcggg ttctggtacg   1620
   attgatgacg acgacaagag tccgggagct cgtggatccg aattc                    1665
<210> 17
   <211> 555
   <212> PRT
   <213> Artificial sequence
<220>
   <223> Protein sequence encoding NusA
<400> 17
   Met Asn Lys Glu Ile Leu Ala Val Val Glu Ala Val Ser Asn Glu Lys
   1               5                   10                  15
   Ala Leu Pro Arg Glu Lys Ile Phe Glu Ala Leu Glu Ser Ala Leu Ala
               20                  25                  30
   Thr Ala Thr Lys Lys Lys Tyr Glu Gln Glu Ile Asp Val Arg Val Gln
           35                  40                  45
   Ile Asp Arg Lys Ser Gly Asp Phe Asp Thr Phe Arg Arg Trp Leu Val
       50                  55                  60
   Val Asp Glu Val Thr Gln Pro Thr Lys Glu Ile Thr Leu Glu Ala Ala
   65                  70                  75                  80
   Arg Tyr Glu Asp Glu Ser Leu Asn Leu Gly Asp Tyr Val Glu Asp Gln
                   85                  90                  95
   Ile Glu Ser Val Thr Phe Asp Arg Ile Thr Thr Gln Thr Ala Lys Gln
               100                 105                 110
   Val Ile Val Gln Lys Val Arg Glu Ala Glu Arg Ala Met Val Val Asp
   115 120 125
   Gln Phe Arg Glu His Glu Gly Glu Ile Ile Thr Gly Val Val Lys Lys
   130 135 140
   Val Asn Arg Asp Asn Ile Ser Leu Asp Leu Gly Asn Asn Ala Glu Ala
   145 150 155 160
   Val Ile Leu Arg Glu Asp Met Leu Pro Arg Glu Asn Phe Arg Pro Gly
   165 170 175
   Asp Arg Val Arg Gly Val Leu Tyr Ser Val Arg Pro Glu Ala Arg Gly
   180 185 190
   Ala Gln Leu Phe Val Thr Arg Ser Lys Pro Glu Met Leu Ile Glu Leu
   195 200 205
   Phe Arg Ile Glu Val Pro Glu Ile Gly Glu Glu Val Ile Glu Ile Lys
   210 215 220
   Ala Ala Ala Arg Asp Pro Gly Ser Arg Ala Lys Ile Ala Val Lys Thr
   225 230 235 240
   Asn Asp Lys Arg Ile Asp Pro Val Gly Ala Cys Val Gly Met Arg Gly
   245 250 255
   Ala Arg Val Gln Ala Val Ser Thr Glu Leu Gly Gly Glu Arg Ile Asp
   260 265 270
   Ile Val Leu Trp Asp Asp Asn Pro Ala Gln Phe Val Ile Asn Ala Met
   275 280 285
   Ala Pro Ala Asp Val Ala Ser Ile Val Val Asp Glu Asp Lys His Thr
   290 295 300
   Met Asp Ile Ala Val Glu Ala Gly Asn Leu Ala Gln Ala Ile Gly Arg
   305 310 315 320
   Asn Gly Gln Asn Val Arg Leu Ala Ser Gln Leu Ser Gly Trp Glu Leu
   325 330 335
   Asn Val Met Thr Val Asp Asp Leu Gln Ala Lys His Gln Ala Glu Ala
   340 345 350
   His Ala Ala Ile Asp Thr Phe Thr Lys Tyr Leu Asp Ile Asp Glu Asp
   355 360 365
   Phe Ala Thr Val Leu Val Glu Glu Gly Phe Ser Thr Leu Glu Glu Leu
   370 375 380
   Ala Tyr Val Pro Met Lys Glu Leu Leu Glu Ile Glu Gly Leu Asp Glu
   385 390 395 400
   Pro Thr Val Glu Ala Leu Arg Glu Arg Ala Lys Asn Ala Leu Ala Thr
   405 410 415
   Ile Ala Gln Ala Gln Glu Glu Ser Leu Gly Asp Asn Lys Pro Ala Asp
   420 425 430
   Asp Leu Leu Asn Leu Glu Gly Val Asp Arg Asp Leu Ala Phe Lys Leu
           435                 440                 445
   Ala Ala Arg Gly Val Cys Thr Leu Glu Asp Leu Ala Glu Gln Gly Ile
       450                 455                 460
   Asp Asp Leu Ala Asp Ile Glu Gly Leu Thr Asp Glu Lys Ala Gly Ala
   465                 470                 475                 480
   Leu Ile Met Ala Ala Arg Asn Ile Cys Trp Phe Gly Asp Glu Ala Thr
                   485                 490                 495
   Ser Gly Ser Gly His His His His His His Ser Ala Gly Lys Glu Thr
               500                 505                 510
   Ala Ala Ala Lys Phe Glu Arg Gln His Met Asp Ser Pro Pro Pro Thr
           515                 520                 525
   Gly Leu Val Pro Arg Gly Ser Ala Gly Ser Gly Thr Ile Asp Asp Asp
       530                 535                 540
   Asp Lys Ser Pro Gly Ala Arg Gly Ser Glu Phe
   545                 550                 555